# EUROPEAN PATENT APPLICATION

(11) **EP 4 202 394 A1**
(43) Date of publication of application: **28.06.2023**
(21) Application number: 22213341.5
(22) Date of filing: 09.11.2017
(51) Int. Cl.: G01M 3/04, D01F 4/00, A61F 13/04

(54) **USE OF A SHRINKABLE BIOPOLYMER FIBER AS SENSOR**

(30) Priority: 11.11.2016 EP 16198399
(62) Divisional of application: 17801636.6
(71) Applicant: AMSilk GmbH, 82061 Neuried (DE)
(72) Inventor: KLEIN, Jens, 82049 Pullach i. Isartal (DE); RÖMER, Lin, 85521 Ottobrunn (DE); BERGFELD, Michael, 81369 München (DE); LAUER, Josef, 80689 München (DE)
(74) Representative: Geling, Andrea

(57) **Abstract**

The present invention relates to the use of a shrinkable biopolymer fiber as sensor. In a first embodiment, the sensor allows to determine the authenticity of a product. In a second embodiment, the sensor allows to determine the presence of a solvent. Further, the present invention relates to a method for determining the authenticity of a product. Furthermore, the present invention relates to a method for determining the presence of a solvent. In addition, the present invention relates to the use of a shrinkable biopolymer fiber for shaping an object. Moreover, the present invention relates to a method for shaping an object. Moreover, the present invention relates to the use of a shrinkable biopolymer fiber as suture material or wound dressing.

## Description

The present invention relates to the use of a shrinkable biopolymer fiber as sensor. In a first embodiment, the sensor allows to determine the authenticity of a product. In a second embodiment, the sensor allows to determine the presence of a solvent. Further, the present invention relates to a method for determining the authenticity of a product. Furthermore, the present invention relates to a method for determining the presence of a solvent. In addition, the present invention relates to the use of a shrinkable biopolymer fiber for shaping an object. Moreover, the present invention relates to a method for shaping an object. Moreover, the present invention relates to the use of a shrinkable biopolymer fiber as suture material or wound dressing.

### BACKGROUND OF THE INVENTION

Counterfeit consumer goods are a common problem in this days. Counterfeit consumer goods are goods made or sold under another's brand name without the brand owner's authorization. They exist in virtually every area, including food, beverages, clothes, shoes, pharmaceuticals, electronics, auto parts, toys, and currency. The spread of counterfeit goods is worldwide, and in 2008 a study by the International Chamber of Commerce (ICC) estimated the global value of all counterfeit goods reached $650 billion every year. The same study projected that in 2015 the upper bound of the global value of counterfeit and pirated goods could be $1.77 trillion. Counterfeit clothes, shoes, jewelry, and handbags from designer brands are made in varying quality; sometimes the intent is only to fool the gullible buyer who only looks at the label or hangtag and does not know what the real thing looks like, while others put some serious effort into mimicking fashion details. Others realize that most consumers do not care if the goods they buy are counterfeit and just wish to purchase inexpensive products. A plurality of counterfeit products are made in such a way that they are difficult to distinguish from the original. This is particularly a problem for the sellers of such products. Sellers of such products may infringe on either the trade mark, patent or copyright of the brand owner by passing off its goods as made by the brand owner.

Packaging can be engineered to help reduce the risks of package pilferage or the theft and resale of products: Some package constructions are more resistant to pilferage and some have pilfer indicating seals. Counterfeit consumer goods, unauthorized sales (diversion), material substitution and tampering can all be reduced with these anti-counterfeiting technologies. Packages may include authentication seals and use security printing to help indicate that the package and contents are not counterfeit; these too are subj ect to counterfeiting. Packages can also include anti-theft devices, such as dye-packs, RFID tags, or electronic article surveillance tags that can be activated or detected by devices at exit points and require specialized tools to deactivate. Anti-counterfeiting technologies that can be used with packaging include, for example, taggant fingerprinting (uniquely coded microscopic materials that are verified from a database), encrypted micro-particles (unpredictably placed markings (numbers, layers, and colors) not visible to the human eye), UV printing (marks only visible under UV light) or serialized barcodes.

Anti-counterfeiting technologies which are currently on the marked have the disadvantage that they are partially non-effective, expensive, or unwieldy for practical use.

Therefore, there is further need for an effective, inexpensive, and a user-friendly system that can be used to determine the authenticity of a product.

Moreover, in many production segments it is important to ensure the quality of a product. A reduced product quality can be a serious public health and safety problem. In addition, a reduced product quality may have a negative impact on the functional capability of a product. One of the most common events leading to reduced product quality is the entry of liquid/moisture into a product or that a product becomes wet. For example, a drug which has become wet does not have an adequate quality anymore. It may be ineffective in treatment. In addition, an electronical device which has become wet does not have an adequate quality anymore. It may function insufficiently or may not function anymore.

Liquid/moisture sensors/indicators which are currently on the market indicate, for example, the liquid/moisture stage though a change in color if a specific liquid/moisture level is exceeded. The color reaction depends on the absorption of water. Most known is cobalt chloride blended silica-gel. It changes its color from violet to pink. Cobalt chloride is, however, toxic to the user/consumer. Alternative non-toxic systems are often expensive.

Thus, there is a further need for an effective, inexpensive, and a non-toxic system that can be used to determine the presence of liquid/moisture.

The inventors of the present patent application surprisingly found that the ability of a biopolymer fiber to shrink allows its use as a sensor. They noticed that said shrinkable biopolymer fiber allows on the one hand side to determine the authenticity of a product and on the other hand side the presence of a solvent in an effective, inexpensive, and user friendly way.

### SUMMARY OF THE INVENTION

In a first aspect, the present invention relates to the use of a shrinkable biopolymer fiber as sensor. In a first embodiment, the sensor allows to determine the authenticity of a product. In a second embodiment, the sensor allows to determine the presence of a solvent.

In a second aspect, the present invention relates to a method for determining the authenticity of a product comprising the steps of:
(i) providing a shrinkable biopolymer fiber as sensor,
(ii) contacting said fiber with a solvent, and
(iii) observing whether a shrinkage of said fiber after contact with the solvent occurs,
   wherein a shrinkage of at least 10% with regard to the total length of said fiber is indicative for the authenticity of the product.

In a third aspect, the present invention relates to a method for determining the presence of a solvent comprising the steps of:
(i) providing a shrinkable biopolymer fiber as sensor, and
(ii) observing whether a shrinkage of said fiber occurs,
   wherein a shrinkage of at least 10% with regard to the total length of said fiber is indicative for the presence of a solvent in contact with said fiber.

This summary of the invention does not necessarily describe all features of the present invention. Other embodiments will become apparent from a review of the ensuing detailed description.

In a fourth aspect, the present invention relates to the use of a shrinkable biopolymer fiber for shaping an object.

In a fifth aspect, the present invention relates to a method for shaping an object comprising the steps of:
(i) providing an object comprising or consisting of a shrinkable biopolymer fiber, and
(ii) contacting said object with a solvent, thereby shaping the object.

In a sixth aspect, the present invention relates to the use of a shrinkable biopolymer fiber as suture material.

In a seventh aspect, the present invention relates to the use of a shrinkable biopolymer fiber as wound dressing.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of" according to the present invention means the inclusion of a stated integer or group of integers, while excluding modifications or other integers which would materially affect or alter the stated integer. The term "consisting of" or variations such as "consists of" according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "sensor", as used herein, refers to an object/a material whose capability is to detect events or changes in its environment and, then, provide a corresponding output. More specifically, the term "sensor", as used herein, refers to an object/a material that reacts after contact with a liquid or moisture. This reaction is optically recognizable. It is expressed in a shape change, preferably a shrinkage.

The term "biopolymer", as used herein, refers to a large molecule or macromolecule of biological origin which is composed of many repeated subunits/repeating building blocks. The biopolymer may be a polypeptide, e.g. a recombinant polypeptide. Said polypeptide comprises repeated subunits/repeating building blocks made of amino acids. Preferably, the polypeptide is a silk polypeptide, more preferably a spider silk polypeptide. An exemplarily process for producing a biopolymer which may be used in the present invention is described in WO 2006/008163.

The terms "polypeptide" and "protein" are used interchangeably in the context of the present invention. They refer to a long peptide-linked chain of amino acids, e.g. one that is at least 40 amino acids long.

The term "silk polypeptide", as used herein, refers to a polypeptide which shows, in comparison to other polypeptides, a quite aberrant amino acid composition. In particular, a silk polypeptide possess large quantities of hydrophobic amino acids such as glycine or alanine, but, for example, no (or only very little) tryptophan. In addition, a silk polypeptide contains highly repetitive amino acid sequences or repetitive units (repeat units, modules), especially in their large core domain.

Based on DNA analysis, it was shown that all silk polypeptides are chains of repetitive units which further comprise a limited set of distinct shorter peptide motifs. The expressions "peptide motif" and "consensus sequence" can be used interchangeably herein. Generally, the silk consensus sequences can be grouped into four major categories: GPGXX, GGX, Aₓ or (GA)ₙ and spacers. These categories of peptide motifs in silk polypeptides have been assigned structural roles. For example, it has been suggested that the GPGXX motif is involved in a β-turn spiral, probably providing elasticity. The GGX motif is known to be responsible for a glycine-rich 3i-helix. Both GPGXX and GGX motifs are thought to be involved in the formation of an amorphous matrix that connects crystalline regions, thereby providing elasticity of the fiber. Alanine-rich motifs typically contain 6-9 residues and have been found to form crystalline β-sheets. The spacers typically contain charged groups and separate the iterated peptide motifs into clusters. Preferably, the silk polypeptide is a spider silk polypeptide. More preferably, the silk polypeptide, e.g. spider silk polypeptide, is a recombinant polypeptide.

The term "biopolymer fiber", as used herein, refers to a substance that is significantly longer than it is wide. It comprises or consists of biopolymers.

The term "biopolymer fiber", as used herein, further refers to a material comprising or consisting of biopolymers that are continuous filaments, staple fibers, or are in discrete elongated pieces.

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0,5 µm and 300 µm. It is more preferred that the biopolymer fiber has a thickness (diameter) of between 1 µm and 200 µm.

The term "biopolymer fiber", as used herein, also encompasses a monofilament or multifilament biopolymer fiber. A monofilament biopolymer fiber is composed of a single (mono)filament. A multifilament biopolymer fiber is composed of a number of (mono)filaments. For example, a multifilament fiber may be composed of between 2 and 1000 (mono)filaments, e.g. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 200, 300, 400, 500, 600, 700, 800, 900, or 1000 (mono)filaments.

The term "biopolymer fiber", as used herein, further encompasses single-drawn and multi-drawn (e.g. double-drawn) fibers. Said fibers have been stretched one or more times during their preparation process, in particular wet-spinning process. An exemplarily process for processing a biopolymer into fibers which may be used in the present invention is described in WO 2014/037453.

The term "biopolymer fiber", as used herein, also covers a biopolymer fiber which comprises/is blended with one or more additional polymers such as additional biopolymers and/or additional plastic polymers. For example, the biopolymer fiber may comprise/may be blended with one or more additional biopolymers selected from the group consisting of a (recombinant) silk polypeptide (e.g. a (recombinant) spider silk polypeptide, a(n) (recombinant) insect silk polypeptide, and/or a (recombinant) silk worm *(Bombyx mori)* silk polypeptide), collagen (e.g. natural and/or recombinant collagen), keratin, and polyolefins (e.g. polylactic acid (PLA), polycaprolactone (PCL), polylactat (PLA), and/or polyhydroxybutyrate (PHB)). The additional plastic polymer may be polyacrylate. In a particular example, (i) the (recombinant) silk polypeptide fiber may comprise a (recombinant) silk polypeptide as biopolymer and collagen as additional biopolymer, (ii) the (recombinant) silk polypeptide fiber may comprise a (recombinant) silk polypeptide as biopolymer and keratin as additional biopolymer, (iii) the (recombinant) spider silk polypeptide fiber may comprise a (recombinant) spider silk polypeptide as biopolymer and a(n) (recombinant) insect silk polypeptide as additional biopolymer, (iv) the (recombinant) spider silk polypeptide fiber may comprise a (recombinant) spider silk polypeptide as biopolymer and a (recombinant) silk worm *(Bombyx mori)* silk polypeptide as additional biopolymer, or (v) the (recombinant) spider silk polypeptide fiber may comprise a (recombinant) spider silk polypeptide as biopolymer and another/different (recombinant) spider silk polypeptide as additional biopolymer.

Preferably, the content of the one or more additional polymers such as biopolymers and/or plastic polymers in the biopolymer fiber is less than 66% by weight, more preferably less than 50% by weight, less than 30% by weight, or less than 20% by weight, and even more preferably less than 15% by weight, less than 10% by weight, less than 5% by weight, or less than 1% by weight. Alternatively, it is preferred that the content of the one or more additional polymers in the biopolymer fiber is at least 0.1% by weight, at least 1% by weight, at least 5% by weight, at least 10% by weight, at least 15% by weight, at least 20% by weight, at least 30% by weight, or at least 50% by weight, and/or less than 66% by weight, less than 50% by weight, less than 30% by weight, less than 20% by weight, less than 10% by weight, less than 5% by weight, or less than 1% by weight. It is, thus, particularly preferred that the content of the one or more additional polymers in the biopolymer fiber is in the range of between 0.1 and 66% by weight, between 1% and 66% by weight, between 5% and 50% by weight, between 5% and 30% by weight, between 5% and 20% by weight, or between 5% and 10% by weight. Such biopolymer fibers may enhance any desired characteristics, e.g. appearance, softness, weight, durability, water-repellent properties, improved cost-of-manufacture, Said characteristics may be useful in medical, industrial, or commercial applications.

The term "shrinkable biopolymer fiber", as used herein, refers to a biopolymer fiber the length of which is shrinkable/reducible after first contact with a chemical, e.g. a solvent. In this respect, it should be noted that the entire shrinkage process (which starts with the first contact with a chemical, e.g. a solvent, and ends with the conclusion of the shrinkage) completely takes place in the chemical, e.g. the solvent. Preferably, the length of the biopolymer fiber is shrinkable/reducible by at least 10%, more preferably by at least 15%, even more preferably by at least 25%, and most preferably by at least 35%, with regard to its total length after first contact with a chemical, e.g. a solvent.

The term "shrinkable biopolymer fiber", as used herein, also refers to a biopolymer fiber which shrinkage (process) starts after a duration after first contact with a chemical, e.g. a solvent, and is concluded after a time range. In this respect, it should be noted that the entire shrinkage process (which starts after a duration after first contact with a chemical, e.g. a solvent, and ends with the shrinkage conclusion after a time range) completely takes place in the chemical, e.g. the solvent. Preferably, the length of the biopolymer fiber is shrinked/reduced by at least 10%, more preferably by at least 15%, even more preferably by at least 25%, and most preferably by at least 35%, with regard to its total length after first contact with the chemical, e.g. the solvent. The fiber may be a fiber as produced in WO 2014/037453.

The term "solvent", as used herein, refers to an aqueous solution or a solution comprising alcohol. It is preferred that the aqueous solution is a buffered aqueous solution, such as Tris/HCl, or water (H₂O), such as technical H₂O or deionized H₂O. In another preferred embodiment the water may be piped water, rainwater, or seawater. It is also preferred that the alcohol is ethanol or isopropanol.

The term "after first contact with a chemical" in this context means that the biopolymer fiber as described herein is, after its production/formation, for the first time contacted with a chemical, e.g. a solvent. The contact with a chemical, e.g. a solvent, preferably results in a shrinkage of at least 10%, more preferably of at least 15%, even more preferably of at least 25%, and most preferably of at least 35%, with regard to its total length. Preferably, the shrinkage is irreversible.

The term "irreversible shrinkage", as used herein, means that the fiber does not return to its original shape without external impacts/influences, in particular without a force application, e.g. fiber extrusion, fiber extension, or fiber stretching.

The term "product", as used herein, refers to anything that can be offered to a market that might satisfy a want or need. The product may be bought as raw material or sold as finished good. The product may be a pharmaceutical product (e.g. a drug), a cosmetical product, an electronical product, a mechanical product, bags (e.g. handbags), footwear or clothing. With respect to the authenticity proof, the product is preferably a fabric, e.g. a woven fabric or knitted fabric. More preferably, the fabric, e.g. the woven fabric or knitted fabric, is a garment.

The term "authenticity of a product", as used herein, refers to the determination whether a product is an original and not copied or an imitation.

The term "label" or "hangtag", as used herein, refers to a piece of polymer, e.g. cloth, which is affixed/attached to a product or is part of a product, or which is affixed/attached to the packaging of a product or part of the packaging comprising a product. It usually comprises written or printed information about the product. The biopolymer fiber as described herein is preferably part of the label or hangtag.

The term "object", as used herein, refers to any object which may be shaped by the shrinkable biopolymer fiber. The object comprises or consists of a shrinkable biopolymer fiber. In one embodiment, the object comprises or consists of one single shrinkable biopolymer fiber. In another embodiment, the object comprises or consists of two or more shrinkable biopolymer fibers.

It is preferred that the object is a garment, apparel, a medical object, an orthopaedic object, a sports equipment including footwear, an outdoor equipment including footwear. The term "object" also covers footwear. It is also preferred that the object is a fabric, e.g. a woven fabric or knitted fabric. It is particularly preferred that the fabric, e.g. the woven fabric or knitted fabric, is a garment. The garment may be a fashion, a sport, an outdoor, a medical, or an orthopaedic garment. The garment may be fashion articles, fashion goods, shirts, socks, stockings, e.g. compression stockings, medical stockings, or support stockings, tights, e.g. support tights, pants, e.g. sport or outdoor pants, underwear, e.g. sport or outdoor underwear, gloves, caps, storm hoods, footwear or bandages.

The term "object", as used herein, covers objects which comprise one or more biopolymer fibers and one or more additional polymer fibers such as additional biopolymer fibers or additional plastic polymer fibers. For example, the object may comprise additional biopolymer fibers selected from the group consisting of (recombinant) silk polypeptide fibers (e.g. (recombinant) spider silk polypeptide fibers, (recombinant) insect silk polypeptide fibers, and/or (recombinant) silk worm *(Bombyx mori)* silk polypeptide fibers), collagen fibers (e.g. natural fibers and/or recombinant collagen fibers), keratin fibers, and polyolefins fibers (e.g. polylactic acid (PLA) fibers, polycaprolactone (PCL) fibers, polylactat (PLA) fibers, and/or polyhydroxybutyrate (PHB)) fibers). The additional plastic polymer fiber may be a polyacrylate fiber.

In a particular example, the object may comprise (i) (recombinant) silk polypeptide fibers as biopolymer fibers and collagen fibers as additional biopolymer fibers, (ii) (recombinant) silk polypeptide fibers as biopolymer fibers and keratin fibers as additional biopolymer fibers, (iii) (recombinant) spider silk polypeptide fibers as biopolymer fibers and (recombinant) insect silk polypeptide fibers as additional biopolymer fibers, or (iv) (recombinant) spider silk polypeptide fibers as biopolymer fibers and a (recombinant) silk worm *(Bombyx mori)* silk polypeptide fibers as additional biopolymer fibers.

Preferably, the object is to at least 5%, at least 10%, at least 20%, at least 30%, at least 40%, at least 50%, at least 60%, at least 70%, at least 80%, at least 90%, at least 95%, at least 99%, or to 100% composed of (a) shrinkable biopolymer fiber(s) and/or the object is to at least 95%, at least 90%, at least 80%, at least 70%, at least 60%, at least 50%, at least 40%, at least 30%, at least 20%, at least 10%, at least 5%, or at least 1% composed of one or more additional polymer fibers such as additional biopolymer fibers or additional plastic polymer fibers.

More preferably, the object is to at least 50%, at least 60%, at least 70%, at least 80%, at least 90%, at least 95%, at least 99%, or to 100% composed of (a) shrinkable biopolymer fiber(s) and/or the object is to at least 50%, at least 40%, at least 30%, at least 20%, at least 10%, at least 5%, or at least 1% composed of one or more additional polymer fibers such as additional biopolymer fibers or additional plastic polymer fibers.

The term "shaping an object", as used herein, encompasses, for example, shrinking an object, compressing an object, and/or reducing the size of an object.

The term "suture material", as used herein, refers to a material used to hold body tissues or body vessels together after injury or surgery. In a preferred embodiment, the suture material is a suture, e.g. a surgical suture. Applications of suture materials generally involve the use of a needle with an attached length of tread or fiber. A number of different shapes, sizes, and thread material have been developed. Surgeons, physicians, dentist, podiatrists, eye doctors, registered nurses and other trained nursing personnel, medics, and clinical pharmacists typically engage in suturing. Surgical knots are used to secure sutures.

The term "wound dressing", as used herein, refers to a pad or compress, in particular in a sterile form, to promote wound healing and protect the wound from further harm. A wound dressing is designed to be in direct contact with the wound. In a preferred embodiment, the wound dressing is self-adhesive.

### Embodiments of the invention

The inventors of the present patent application surprisingly found that the ability of a biopolymer fiber to shrink allows its use as a sensor. They noticed that said shrinkable biopolymer fiber allows on the one hand side to determine the authenticity of a product and on the other hand side the presence of a solvent in an effective, inexpensive, and user friendly way.

Thus, in a first aspect, the present invention relates to the use of a shrinkable biopolymer fiber as sensor. Said sensor may be an authenticity sensor or a liquid/moisture sensor.

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

Particularly, the biopolymer fiber has a specific shrinkage behavior. Said specific shrinkage behavior is influenceable/influenced by temperature and/or pH. More particularly, the shrinkage (process) starts after first contact with a solvent/after first contact of a solvent with said fiber. Even more particularly, the shrinkage (process) starts after a duration after first contact with a solvent/after first contact of a solvent with said fiber and is concluded after a time range. The inventors of the present patent application surprisingly found that an increase in temperature/a temperature increase reduces the duration between first contact of a solvent/first contact of a solvent with said fiber and start of the shrinkage/shrinkage process ("time to shrink"), and/or that an increase in temperature/a temperature increase reduces the time range in which the shrinkage is concluded ("shrinkage duration"). In addition, the inventors of the present patent application surprisingly found that an increase in pH (towards a more basic pH) reduces the time range in which the shrinkage (process) is concluded ("shrinkage duration"). In this respect, it should be noted that the entire shrinkage process (which starts after a duration after first contact with a chemical, e.g. a solvent, and ends with the shrinkage conclusion after a time range) completely takes place in the chemical, e.g. the solvent.

In a preferred embodiment the fiber is coated/finished. The inventors of the present patent application surprisingly found that the coating may effect the shrinkage behavior. A coating/finishing may prolong the duration between first contact of a solvent/first contact of a solvent with said fiber and start of the shrinkage/shrinkage process ("time to shrink"), and/or prolong the time range in which the shrinkage is concluded ("shrinkage duration").

It is preferred that the shrinkage (process) takes place at a temperature of between 8°C and 37°C, e.g. between 15°C and 25°C. In other words, it is preferred that the temperature at the time of contacting the shrinkable biopolymer fiber with the solvent or the solvent with the shrinkable biopolymer fiber is between 8 and 37°C, e.g. between 8 and 35°C, 15 and 25°C, 16 and 24°C, or 24 and 35°C. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C.

It is alternatively or additionally preferred that the shrinkage (process) takes place at a pH of between 2.8 and 12.2, e.g. between pH 6 and 8. In other words, it is preferred that the pH of the solvent is between pH 2.8 and 12.2, e.g. between pH 6 and 8. For example, the pH may be pH 2.8, 2.9, 3, 4, 5, 6, 7, 8, 9, 10, 11, 12, 12.1, or 12.2.

In a first embodiment, the sensor allows to determine the authenticity of a product.

It is preferred that said fiber shows a shrinkage of at least 10% with regard to its total length after first contact with a solvent. It is more preferred that said fiber shows a shrinkage of at least 15% with regard to its total length after first contact with a solvent. It is even more preferred that said fiber shows a shrinkage of at least 25% with regard to its total length after first contact with a solvent. It is most preferred that said fiber shows a shrinkage of at least 35% with regard to its total length after first contact with a solvent. For example, said fiber shows a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to its total length after first contact with a solvent. It is also preferred that said fiber shows a shrinkage of between 10% and 50% with regard to its total length after first contact with a solvent. It is more preferred that said fiber shows a shrinkage of between 15% and 35% with regard to its total length after first contact with a solvent. It is even more preferred that said fiber shows a shrinkage of between 15% and 25% with regard to its total length after first contact with a solvent. For example, said fiber shows a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to its total length after first contact with a solvent.

During shrinkage, the cross-sectional area of the biopolymer fiber increases.

Preferably, the shrinkage is indicative for the authenticity of a product.

It is further preferred that the shrinkage (process) starts between 3 and 200 seconds after first contact with the solvent. It is more preferred that the shrinkage (process) starts between 4 and 120 seconds after first contact with the solvent. It is even more preferred that the shrinkage (process) starts between 20 and 60 seconds after first contact with the solvent. It is most preferred that the shrinkage (process) starts between 20 and 50 seconds after first contact with the solvent. For example, the shrinkage (process) starts between 3 and 200 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, between 17 and 47 seconds, after first contact with the solvent. The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be, 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage (process) starts between 4 and 120 seconds after first contact with the solvent at a temperature of between 8 and 35°C, and/or at a pH of between pH 2.8 and 12.2. It is more preferred that the shrinkage (process) starts between 20 and 60 seconds after first contact with the solvent at a temperature of between 16 and 24°C, and/or at a pH of between pH 2.8 and 12.2. It is even more preferred that the shrinkage (process) starts between 4 and 25 seconds after first contact with the solvent at a temperature of between 24 and 35 °C, and/or at a pH of between pH 2.8 and 12.2. It is most preferred that the shrinkage (process) starts between 25 and 45 seconds after first contact with the solvent at a temperature of between 16 and 24°C, and/or at a pH of between pH 6 and 8.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)). The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 550 seconds (after start of the shrinkage (process)) at a temperature of between 8 and 35°C, and/or at a pH of between pH 2.8 and 12.2. It is more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 110 and 275 seconds (after start of the shrinkage (process)) at a temperature of between 16 and 24°C, and/or at a pH of between pH 2.8 and 12.2. It is even more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 150 seconds (after start of the shrinkage (process)) at a temperature of between 24 and 35°C, and/or at a pH of between pH 2.8 and 12.2. It is most preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 115 and 230 seconds (after start of the shrinkage (process)) at a temperature of between 16 and 24°C, and/or at a pH of between pH 6 and 8.

Preferably, the shrinkage is irreversible. As mentioned above, irreversible means that the fiber does not return to its original shape without external impact/influences, in particular without force application, e.g. fiber extrusion, fiber extension, or fiber stretching. It is generally possible to re-extend the shrinked biopolymer fiber nearly to its original shape by techniques known to the person skilled in the art, e.g. by fiber extrusion, fiber extension, or fiber stretching. It is then possible to shrink the fiber by contacting said fiber with a solvent for a second time/again. However, the inventors of the present invention noticed that the shrinkage behavior of such a biopolymer fiber differs from a biopolymer fiber which shrinks after a first contact with a solvent.

The solvent may be an aqueous solution or a solution comprising alcohol. It is preferred that the aqueous solution is a buffered aqueous solution, such as Tris/HCl, or water (H₂O), such as technical H₂O or deionized H₂O. It is also preferred that the alcohol is ethanol or isopropanol.

Preferably, the product is a fabric, e.g. a woven fabric or knitted fabric. More preferably, the fabric, e.g. the woven fabric or knitted fabric, is a garment.

Preferably, the shrinkable biopolymer fiber is part of a label or hangtag. The label or hangtag is a piece of cloth. It is clear for the skilled person that the label or hangtag comprises more than one fiber. It is, in fact, composed of a plurality of fibers. In other words, the label or hangtag comprises or consists of fibers. The label or hangtag may be a woven cloth. In this case, it is clear for the skilled person that the label or hangtag comprises or consists of fibers which are weaved to each other. It will also be understood by the skilled person that the shrinkage in a single biopolymer fiber takes place one-dimensional. In contrast thereto, the shrinkage in a woven cloth comprising or consisting of biopolymer fibers which are weaved to each other takes place multidimensional.

The label or hangtag may further comprise written or printed information about the product.

The label or hangtag may be affixed/attached to the product or is part of the product. If the label or hangtag is part of the product, it is, for example, supplied with the product. Alternatively, the label or hangtag is affixed/attached to the packaging or is part of the packaging comprising the product. That means that the label or hangtag is not directly affixed/attached to the product itself but affixed/attached to the overpack with which the product is distributed/sold.

In a second embodiment, the sensor allows to determine the presence of a solvent/the presence of a solvent at any time in the past, e.g. water. The water may be piped water, rainwater, or seawater.

It is preferred that said fiber shows a shrinkage of at least 10% with regard to its total length after first contact with a solvent. It is more preferred that said fiber shows a shrinkage of at least 15% with regard to its total length after first contact with a solvent. It is even more preferred that said fiber shows a shrinkage of at least 25% with regard to its total length after first contact with a solvent. It is most preferred that said fiber shows a shrinkage of at least 35% with regard to its total length after first contact with a solvent. For example, said fiber shows a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to its total length after first contact with a solvent. It is also preferred that said fiber shows a shrinkage of between 10% and 50% with regard to its total length after first contact with a solvent. It is more preferred that said fiber shows a shrinkage of between 15% and 35% with regard to its total length after first contact with a solvent. It is even more preferred that said fiber shows a shrinkage of between 15% and 25% with regard to its total length after first contact with a solvent. For example, said fiber shows a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to its total length after first contact with a solvent.

During shrinkage, the cross-sectional area of the biopolymer fiber increases.

Preferably, the shrinkage is indicative for the presence of a solvent/the presence of a solvent at any time in the past, e.g. water. The water may be piped water, rainwater, or seawater.

It is further preferred that the shrinkage (process) starts between 3 and 200 seconds after first contact with the solvent. It is more preferred that the shrinkage (process) starts between 4 and 120 seconds after first contact with the solvent. It is even more preferred that the shrinkage (process) starts between 20 and 60 seconds after first contact with the solvent. It is most preferred that the shrinkage (process) starts between 20 and 50 seconds after first contact with the solvent. For example, the shrinkage (process) starts between 3 and 200 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after first contact with the solvent. The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be, 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage (process) starts between 4 and 120 seconds after first contact with the solvent at a temperature of between 8 and 35°C, and/or at a pH of between pH 2.8 and 12.2. It is more preferred that the shrinkage (process) starts between 20 and 60 seconds after first contact with the solvent at a temperature of between 16 and 24°C, and/or at a pH of between pH 2.8 and 12.2. It is even more preferred that the shrinkage (process) starts between 4 and 25 seconds after first contact with the solvent at a temperature of between 24 and 35 °C, and/or at a pH of between pH 2.8 and 12.2. It is most preferred that the shrinkage (process) starts between 25 and 45 seconds after first contact with the solvent at a temperature of between 16 and 24°C, and/or at a pH of between pH 6 and 8.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)). The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 550 seconds (after start of the shrinkage (process)) at a temperature of between 8 and 35°C, and/or at a pH of between pH 2.8 and 12.2. It is more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 110 and 275 seconds (after start of the shrinkage (process)) at a temperature of between 16 and 24°C, and/or at a pH of between pH 2.8 and 12.2. It is even more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 150 seconds (after start of the shrinkage (process)) at a temperature of between 24 and 35°C, and/or at a pH of between pH 2.8 and 12.2. It is most preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 115 and 230 seconds (after start of the shrinkage (process)) at a temperature of between 16 and 24°C, and/or at a pH of between pH 6 and 8.

Preferably, the shrinkage is irreversible. As mentioned above, irreversible means that the fiber does not return to its original shape without external impact/influences, in particular without force application, e.g. fiber extrusion, fiber extension, or fiber stretching. It is generally possible to re-extend the shrinked biopolymer fiber nearly to its original shape by techniques known to the person skilled in the art, e.g. by fiber extrusion, fiber extension, or fiber stretching. It is then possible to shrink the fiber by contacting said fiber with a solvent for a second time/again. However, the inventors of the present invention noticed that the shrinkage behavior of such a biopolymer fiber differs from a biopolymer fiber which shrinks after a first contact with a solvent.

The solvent may be an aqueous solution, e.g. water. The water may be piped water, rainwater, or seawater.

Regarding the first or second embodiment, it is preferred that the biopolymer is a silk polypeptide. It is more preferred that the biopolymer is a recombinant silk polypeptide. The (recombinant) silk polypeptide may be a spider silk polypeptide, e.g. a major ampullate silk polypeptide such as a dragline silk polypeptide, a minor ampullate silk polypeptide, or a flagelliform silk polypeptide of an orb-web spider (e.g. *Araneidae or Araneoids),* an insect silk polypeptide, a mussel byssus silk polypeptide, or a mixture thereof. The orb-web spider may be selected from the group consisting of *Araneus diadematus, Nephila clavipes,* and *Latrodectus hesperus.* The insect silk polypeptide may be of *Lepidoptera,* particularly *Bombycidae* such as *Bombyx mori.* The insect silk polypeptide may also be of *Hymenoptera,* particularly *Apoidea* such as *Anthophila.* Preferably, the silk polypeptide is a spider silk polypeptide, more preferably a recombinant spider silk polypeptide.

It is further (alternatively or additionally) preferred that the silk polypeptide is a polypeptide with an amino acid sequence which comprises or consists of at least 50%, 60%, 65%, 70%, 75%, 80%, 85%, or 90% multiple copies of repetitive units. It is more preferred that the silk polypeptide is a polypeptide with an amino acid sequence which comprises or consists of at least 95% multiple copies of repetitive units. Said repetitive units may be identical or different. It is particularly preferred that the silk polypeptide comprises at least two identical repetitive units. For example, the silk polypeptide may comprise between 2 to 100 repetitive units, e.g. 2, 3, 4, 5, 6, 7, 8, 9, 10, 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 repetitive units.

It is also (alternatively or additionally) preferred that the silk polypeptide consists of between 40 to 3000 amino acids. It is more preferred that the silk polypeptide consists of between 40 to 1500 amino acids. It is even more preferred that the silk polypeptide consists of between 200 to 1200 amino acids. It is most preferred that the silk polypeptide consists of between 250 to 600 amino acids.

As mentioned above, it is particularly preferred that the silk polypeptide comprises at least two identical repetitive units. In one embodiment, the repetitive units are independently selected from the group consisting of module C (SEQ ID NO: 1) or a variant thereof, module C^{Cys} (said module may also be designated as module C^{C}) (SEQ ID NO: 2), and module C^{kappa} (SEQ ID NO: 3). Module C^{Cys} (SEQ ID NO: 2) is a variant of module C (SEQ ID NO: 1). In this module, the amino acid S (Ser) at position 25 has been replaced by the amino acid C (Cys). Module C^{kappa} (SEQ ID NO: 3) is also a variant of module C (SEQ ID NO: 1). In this module, the amino acid E (Glu) at position 20 has been replaced by the amino acid K (Lys).

The module C variant differs from the reference module C from which it is derived by up to 1, 2, 3, 4, 5, 6, 7, 8, 9, 10, 11, 12, 13, 14, or 15 amino acid changes in the amino acid sequence (i.e. substitutions, additions, insertions, deletions, N-terminal truncations and/or C-terminal truncations). Such a module variant can alternatively or additionally be characterised by a certain degree of sequence identity to the reference module from which it is derived. Thus, the module C variant has a sequence identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or even 99.9% to the respective reference module C. Preferably, the sequence identity is over a continuous stretch of at least 5, 10, 15, 18, 20, 24, 27, 28, 30, 34, 35, or more amino acids, preferably over the whole length of the respective reference module C.

The sequence identity may be at least 80% over the whole length, may be at least 85% over the whole length, may be at least 90% over the whole length, may be at least 95% over the whole length, may be at least 98% over the whole length, or may be at least 99% over the whole length of the respective reference module C. Alternatively, the sequence identity may be at least 80% over a continuous stretch of at least 5, 10, 15, 18, 20, 24, 28, or 30 amino acids, may be at least 85% over a continuous stretch of at least 5, 10, 15, 18, 20, 24, 28, or 30 amino acids, may be at least 90% over a continuous stretch of at least 5, 10, 15, 18, 20, 24, 28, or 30 amino acids, may be at least 95% over a continuous stretch of at least 5, 10, 15, 18, 20, 24, 28, or 30 amino acids, may be at least 98% over a continuous stretch of at least 5, 10, 15, 18, 20, 24, 28, or 30 amino acids, or may be at least 99% over a continuous stretch of at least 5, 10, 15, 18, 20, 24, 28, or 30 amino acids of the respective reference module C.

A fragment (or deletion) variant of module C has preferably a deletion of up to 1, 2, 3, 4, 5, 6, 7, 8, 9, 10, 11, 12, 13, 14, or 15 amino acids at its N-terminus and/or at its C-terminus. The deletion can also be internally.

Additionally, the module C variant or fragment is only regarded as a module C variant or fragment within the context of the present invention, if the modifications with respect to the amino acid sequence on which the variant or fragment is based do not negatively affect the ability of the silk polypeptide to act as a sensor. The skilled person can readily assess whether the silk polypeptide comprising a module C variant or fragment is still capable acting as a sensor, e.g. which allows to determine the authenticity of a product or to determine the presence of a solvent. In this respect, it is referred to the shrinkage examples comprised in the experimental part of the present patent application.

C^{Cys} or C^{kappa} variants may also be encompassed by the present invention. Regarding the C^{Cys} or C^{kappa} variants, the same explanations/definitions apply which have been made with respect to the module C variant (see above).

It is further particularly preferred that the silk polypeptide comprises at least one non-repetitive (NR) unit. Said non-repetitive (NR) unit may be comprised at the N- and/or C-terminus. In one embodiment, the NR unit is selected from the group consisting of NR3 (SEQ ID NO: 4) or a variant thereof, NR4 (SEQ ID NO: 5) or a variant thereof, NR5 (SEQ ID NO: 6) or a variant thereof, and NR6 (SEQ ID NO: 7) or a variant thereof. The NR3 (SEQ ID NO: 4) unit is based on the amino acid sequence of ADF-3 of the spider *Araneus diadematus* and the NR4 (SEQ ID NO: 5) unit is based on the amino acid sequence of ADF-4 of the spider *Araneus diadematus* (WO 2006/008163). In addition, the NR5 (SEQ ID NO: 6) unit and the NR6 (SEQ ID NO: 7) unit are derived from *Latrodectus hesperus.*

Regarding the NR3, NR4, NR5, or NR6 unit variant, the same explanations/definitions apply which have been made with respect to the module C variant (see above).

In addition, a NR3, NR4, NR5, or NR6 unit variant or fragment is only regarded as a NR3, NR4, NR5, or NR6 unit variant or fragment within the context of the present invention, if the modifications with respect to the amino acid sequence on which the variant or fragment is based do not negatively affect the ability of the silk polypeptide to act as a sensor. The skilled person can readily assess whether the silk polypeptide comprising a NR3, NR4, NR5, or NR6 unit variant or fragment is still capable acting as a sensor, e.g. which allows to determine the authenticity of a product or to determine the presence of a solvent. In this respect, it is referred to the shrinkage examples comprised in the experimental part of the present patent application.

In one preferred embodiment, the silk polypeptide is selected from the group consisting of (C)ₘ, (C^{CyS})m, (C^{kappa})ₘ, (C)ₘC^{Cys}, C^{Cys}(C)ₘ, (C)ₘC^{CyS}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, c^{kappa}₁₆, C^{kappa}₃₂, C^{kappa}48, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C_{48.}

As mentioned above, the biopolymer fiber has a specific shrinkage behavior. Said shrinkage behavior is further influenceable/influenced by the thickness (diameter) of the biopolymer fiber. More particularly, the shrinkage (process) starts after a duration after first contact with a solvent/after first contact of a solvent with said fiber and is concluded after a time range. The inventors of the present patent application surprisingly found that an increase in the thickness (diameter) of the biopolymer fiber increases the duration between first contact of a solvent/first contact of a solvent with said fiber and start of the shrinkage (process), and/or that an increase in the thickness (diameter) of the biopolymer fiber increases the time range in which the shrinkage is concluded. Preferably, the biopolymer fiber has a thickness (diameter) of between 0.5 µm and 300 µm. More preferably, the biopolymer fiber has a thickness (diameter) of between 1 µm and 200 µm. It is further preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

Considering the aforementioned, the biopolymer fiber has a specific shrinkage behavior which is influenceable/influenced by the following factors: temperature, pH, and/or the thickness (diameter) of the biopolymer fiber. The shrinkage behavior may be influenceable/influenced by additional factors.

Moreover, the inventors of the present patent application surprisingly found that the extent of shrinkage with regard to the total length of the biopolymer fiber can be influenced by the type of biopolymer fiber used as a starting material. In particular, they surprisingly found that the extent of shrinkage with regard to the total length of the biopolymer fiber is influenceable/influenced by the degree of drawing of the biopolymer fiber used as a starting material. As mentioned above, the biopolymer fiber may be a single-drawn or a multi-drawn (e.g. double-drawn) fiber. Said fiber has been stretched one or more times during its preparation process, in particular wet-spinning process. The inventors of the present patent application now observed that the extend of shrinkage with regard to the total length of the biopolymer fiber is higher, the higher the degree of drawing of the biopolymer fiber used as a starting material is. Preferably the extend of shrinkage can be adjusted to the range of 10% and 50% by varying the degree of drawing of the biopolymer fiber during and/or after the spinning process. More preferably the extend of shrinkage can be adjusted to the range of 10% and 35% by varying the degree of drawing of the biopolymer fiber during and/or after the spinning process. For example, the extend of shrinkage is higher with regard to the total length of the biopolymer fiber with a multi-drawn (e.g. double-drawn) biopolymer fiber (e.g. at least 20%) than with a single-drawn biopolymer fiber (e.g. at least 10%).

Based on the above, the biopolymer fiber has a specific shrinkage behavior which is influenceable/influenced by the following factors: temperature, pH, the thickness (diameter) of the biopolymer fiber, and/or the type of the biopolymer fiber used as a starting material (e.g. single-drawn or multi-drawn biopolymer fiber).

The first aspect of the present invention, as described above, can alternatively be worded as follows: In a first aspect, the present invention relates to a sensoring method, wherein a shrinkable biopolymer fiber is used.

In a second aspect, the present invention relates to a method for determining the authenticity of a product comprising the steps of:
(i) providing a shrinkable biopolymer fiber as sensor,
(ii) (first) contacting said fiber with a solvent, and
(iii) observing whether a shrinkage of said fiber after contact with the solvent occurs/occurred,
   wherein a shrinkage of at least 10% with regard to the total length of said fiber is indicative for the authenticity of the product.

As mentioned above, the inventors of the present invention surprisingly found that the ability of a biopolymer fiber to shrink allows its use as a sensor. They noticed that said shrinkable biopolymer fiber allows to determine the authenticity of a product in an effective, inexpensive, and user friendly way.

Preferably, the product is a fabric, e.g. a woven fabric or knitted fabric. More preferably, the fabric, e.g. the woven fabric or knitted fabric, is a garment. The product may also be a bag (e.g. a handbag) or footwear.

In step (i) of the method of the second aspect of the present invention, a shrinkable biopolymer fiber is provided as sensor. It is preferred that the biopolymer is a silk polypeptide. It is more preferred that the biopolymer is a recombinant silk polypeptide. The (recombinant) silk polypeptide may be a spider silk polypeptide, e.g. a major ampullate silk polypeptide such as a dragline silk polypeptide, a minor ampullate silk polypeptide, or a flagelliform silk polypeptide of an orb-web spider (e.g. *Araneidae* or *Araneoids),* an insect silk polypeptide, a mussel byssus silk polypeptide, or a mixture thereof. The orb-web spider may be selected from the group consisting of *Araneus diadematus, Nephila clavipes,* and *Latrodectus hesperus.* The insect silk polypeptide may also be of *Hymenoptera,* particularly *Apoidea* such as *Anthophila.* Preferably, the silk polypeptide is a spider silk polypeptide, more preferably a recombinant spider silk polypeptide.

It is further (alternatively or additionally) preferred that the silk polypeptide is a polypeptide as already described under the first aspect.

It is further particularly preferred that the silk polypeptide comprises at least one non-repetitive (NR) unit as already described under the first aspect. Said non-repetitive (NR) unit may be comprised at the N- and/or C-terminus.

In one preferred embodiment, the silk polypeptide is selected from the group consisting of (C)ₘ, (C^{Cys})m, (C^{kappa})ₘ, (C)ₘC^{Cys}, C^{Cys}(C)ₘ, (C)ₘC^{Cys}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, C^{kappa}₁₆, C^{kappa}₃₂, C^{kappa}48, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C_{48.}

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

Preferably, the shrinkable biopolymer fiber is part of a label or hangtag. The label or hangtag is a piece of cloth. It is clear for the skilled person that the label or hangtag comprises more than one fiber. It is, in fact, composed of a plurality of fibers. In other words, the label or hangtag comprises or consists of fibers. The label or hangtag may be a woven cloth. In this case, it is clear for the skilled person that the label or hangtag comprises or consists of fibers which are weaved to each other.

The label or hangtag may further comprise written or printed information about the product.

The label or hangtag may be affixed/attached to the product or is part of the product. If the label or hangtag is part of the product, it is, for example, supplied with the product. Alternatively, the label or hangtag is affixed/attached to the packaging or is part of the packaging comprising the product. That means that the label or hangtag is not directly affixed/attached to the product itself but affixed/attached to the overpack with which the product is distributed/sold.

In step (ii) of the method of the second aspect of the present invention, said shrinkable biopolymer fiber is/was contacted with a solvent. The contact of the shrinkable biopolymer fiber with the solvent may occur/occurred by dropping the solvent onto said shrinkable biopolymer fiber, by dipping said shrinkable biopolymer fiber into the solvent, or by spraying the solvent onto said shrinkable biopolymer fiber.

As mentioned above, it is preferred that the shrinkable biopolymer fiber is part of a label or hangtag. The label or hangtag comprises or consists of fibers. In this case, the solvent is/was dropped onto the label or hangtag comprising or consisting of fibers, the label or hangtag comprising or consisting of fibers is/was dipped into the solvent, or the solvent is/was sprayed onto the label or hangtag comprising or consisting of fibers. For the contact with the solvent, the label or hangtag may remain on the product or on the packaging comprising the product. Alternatively, the label or hangtag may be removed from the product in order to contact said label or hangtag with a solvent. Depending on the size of the label or hangtag, it may also only partially be removed from the product in order to contact said label or hangtag with a solvent.

The solvent may be an aqueous solution or a solution comprising an alcohol. It is preferred that the aqueous solution is a buffered aqueous solution, such as Tris/HCl, or water (H₂O), such as technical H₂O or deionized H₂O. It is also preferred that the alcohol is ethanol or isopropanol.

In step (iii) of the method of the second aspect of the present invention, it is observed whether a shrinkage of said fiber after contact with the solvent occurs/occurred at any time in the past. Said shrinkage takes place/starts after (first) contact with the solvent in step (ii). The shrinkage is optically recognizable. The shrinkage can also be measured. For example, the length of the fiber may be measured before and after contact with the solvent in order to determine whether a shrinkage occurs/takes place/starts.

In this respect, it should be noted that a shrinkage of at least 10%, preferably of at least 15%, more preferably of at least 25%, even more preferably of at least 35%, with regard to the total length of said fiber is indicative for the authenticity of the product. For example, a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to the total length of said fiber is indicative for the authenticity of the product.

It is also preferred that a shrinkage of between 10% and 50% with regard to the total length of said fiber is indicative for the authenticity of the product. It is more preferred that a shrinkage of between 15% and 35% with regard to the total length of said fiber is indicative for the authenticity of the product. It is even more preferred that a shrinkage of between 15% and 25% with regard to the total length of said fiber is indicative for the authenticity of the product. For example, a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to the total length of said fiber is indicative for the authenticity of the product.

During shrinkage, the cross-sectional area of the biopolymer fiber increases.

It is further preferred that the shrinkage (process) starts between 3 and 200 seconds after (first) contact with the solvent. It is more preferred that the shrinkage (process) starts between 4 and 120 seconds after (first) contact with the solvent. It is even more preferred that the shrinkage (process) starts between 20 and 60 seconds after (first) contact with the solvent. It is most preferred that the shrinkage (process) starts between 20 and 50 seconds after (first) contact with the solvent. For example, the shrinkage (process) starts between 3 and 200 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after (first) contact with the solvent. The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage (process) starts between 4 and 120 seconds after first contact with the solvent at a temperature of between 8 and 35°C, and/or at a pH of between pH 2.8 and 12.2. It is more preferred that the shrinkage (process) starts between 20 and 60 seconds after (first) contact with the solvent at a temperature of between 16 and 24°C, and/or at a pH of between pH 2.8 and 12.2. It is even more preferred that the shrinkage (process) starts between 4 and 25 seconds after (first) contact with the solvent at a temperature of between 24 and 35°C, and/or at a pH of between pH 2.8 and 12.2. It is most preferred that the shrinkage (process) starts between 25 and 45 seconds after (first) contact with the solvent at a temperature of between 16 and 24°C, and/or at a pH of between pH 6 and 8.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)). The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 550 seconds (after start of the shrinkage (process)) at a temperature of between 8 and 35°C, and/or at a pH of between pH 2.8 and 12.2. It is more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 110 and 275 seconds (after start of the shrinkage (process)) at a temperature of between 16 and 24°C, and/or at a pH of between pH 2.8 and 12.2. It is even more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 150 seconds (after start of the shrinkage (process)) at a temperature of between 24 and 35°C, and/or at a pH of between pH 2.8 and 12.2. It is most preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 115 and 230 seconds (after start of the shrinkage (process)) at a temperature of between 16 and 24°C, and/or at a pH of between pH 6 and 8.

Preferably, the shrinkage is irreversible. As mentioned above, irreversible means that the fiber does not return to its original shape without external impact/influences, in particular without force application, e.g. fiber extrusion, fiber extension, or fiber stretching. It is generally possible to re-extend the shrinked biopolymer fiber nearly to its original shape by techniques known to the person skilled in the art, e.g. by fiber extrusion, fiber extension, or fiber stretching. It is then possible to shrink the fiber by contacting said fiber with a solvent for a second time/again. However, the inventors of the present invention noticed that the shrinkage behavior of such a biopolymer fiber differs from a biopolymer fiber which shrinks after a first contact with a solvent.

It will be understood by the skilled person that the shrinkage in a single biopolymer fiber takes place one-dimensional. In contrast thereto, the shrinkage in a woven cloth comprising or consisting of biopolymer fibers which are weaved to each other takes place multidimensional.

Regarding the specific shrinkage behavior of the shrinkable biopolymer fiber which is influenceable/influenced by the following factors: temperature, pH, the thickness (diameter) of the biopolymer fiber, and/or the type of the biopolymer fiber used as a starting material (e.g. single-drawn or multi-drawn biopolymer fiber), it is referred to the first aspect of the present invention.

In a third aspect, the present invention relates to a method for determining the presence of a solvent comprising the steps of:
(i) providing a shrinkable biopolymer fiber as sensor, and
(ii) observing whether a shrinkage of said fiber occurs/occurred,
   wherein a shrinkage of at least 10% with regard to the total length of said fiber is indicative for the presence of a solvent in contact with said fiber.

As mentioned above, the inventors of the present invention surprisingly found that the ability of a biopolymer fiber to shrink allows its use as a sensor. They noticed that said shrinkable biopolymer fiber allows to determine the presence of a solvent in an effective, inexpensive, and user friendly way. In this case, the shrinkable biopolymer fiber acts as a liquid/moisture sensor.

The solvent may be an aqueous solution, e.g. water. The water may be piped water, rainwater, or seawater. The solvent may be present in an area or at a location, e.g. a shipping space, a housing space, an underground garage, or a basement room. The solvent may enter the area or the location, e.g. the shipping space, the housing space, the underground garage, or the basement room. The solvent may also be present in a packaging, e.g. of a product. The solvent may enter this packaging, e.g. of a product. It may reduce the quality of said product or destroy the product. The product may be, for example, a pharmaceutical product, a cosmetical product, an electronical product, or a mechanical product.

In step (i) of the method of the third aspect of the present invention, a shrinkable biopolymer fiber is provided as sensor. It is preferred that the biopolymer is a silk polypeptide. It is more preferred that the biopolymer is a recombinant silk polypeptide. The (recombinant) silk polypeptide may be a spider silk polypeptide, e.g. a major ampullate silk polypeptide such as a dragline silk polypeptide, a minor ampullate silk polypeptide, or a flagelliform silk polypeptide of an orb-web spider (e.g. *Araneidae* or *Araneoids),* an insect silk polypeptide, a mussel byssus silk polypeptide, or a mixture thereof. The orb-web spider may be selected from the group consisting of *Araneus diadematus, Nephila clavipes,* and *Latrodectus hesperus.* The insect silk polypeptide may also be of *Hymenoptera,* particularly *Apoidea* such as *Anthophila.* Preferably, the silk polypeptide is a spider silk polypeptide, more preferably a recombinant spider silk polypeptide.

It is further (alternatively or additionally) preferred that the silk polypeptide is a polypeptide as already described under the first aspect.

It is further particularly preferred that the silk polypeptide comprises at least one non-repetitive (NR) unit as already described under the first aspect.. Said non-repetitive (NR) unit may be comprised at the N- and/or C-terminus

In one preferred embodiment, the silk polypeptide is selected from the group consisting of (C)ₘ, (C^{Cys})m, (C^{kappa})ₘ, (C)ₘC^{Cys}, C^{Cys}(C)ₘ, (C)ₘC^{CyS}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, C^{kappa}₁₆, C^{kappa}₃₂, C^{kappa}48, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C_{48.}

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

Preferably, the shrinkable biopolymer fiber is part of a thread. The thread comprises or consists of shrinkable biopolymer fibers.

In step (ii) of the method of the third aspect of the present invention, it is observed whether a shrinkage of said fiber occurs/takes place/starts. Said shrinkage occurs/takes place/starts after (first) contact of the solvent with said fiber. The shrinkage is optically recognizable. The shrinkage can also be measured. For example, the length of the fiber may be measured before and after contact with the solvent in order to determine whether a shrinkage occurs/takes place/starts. A shrinkage can also be recognized by an electronical or mechanical device which provides an output (e.g. a signal or a change in state) which informs a person (e.g. the user) accordingly. For example, if the shrinkable biopolymer fiber is part of a ship scotland, the shrinkage of said fiber due to the ingress of water (even in small amounts) may be recognized by an electronical or mechanical device which leads to immediate closing of the ship scotland. Alternatively, if the shrinkable biopolymer fiber is part of a ship scotland, the shrinkage of said fiber due to the ingress of water (even in small amounts) may also directly result (without interposition of an electronical or mechanical device) in the immediate closing of the ship scotland. Further, for example, if the shrinkable biopolymer fiber is part of a drug packaging, the shrinkage of said fiber due to the penetration of water informs the person (e.g. the user) that the product has, possibly, not its original quality anymore. Furthermore, for example, if the shrinkable biopolymer fiber is part of a packaging of an electronical or mechanical device, the shrinkage of said fiber due to the entry of water informs the person (e.g. the user) that the product has, possibly, a malfunction and, thus, should not be put into operation.

In this respect, it should be noted that a shrinkage of at least 10%, preferably of at least 15%, more preferably of at least 25%, even more preferably of at least 35%, with regard to the total length of said fiber is indicative for the presence of a solvent in contact with said fiber. For example, a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to the total length of said fiber is indicative for the presence of a solvent in contact with said fiber.

It is also preferred that a shrinkage of between 10% and 50% with regard to the total length of said fiber is indicative for the presence of a solvent in contact with said fiber. It is more preferred that a shrinkage of between 15% and 35% with regard to the total length of said fiber is indicative for the presence of a solvent in contact with said fiber. It is even more preferred that a shrinkage of between 15% and 25% with regard to the total length of said fiber is indicative for the presence of a solvent in contact with said fiber. For example, a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to the total length of said fiber is indicative for the presence of a solvent in contact with said fiber.

During shrinkage, the cross-sectional area of the biopolymer fiber increases.

It is further preferred that the shrinkage (process) starts between 3 and 200 seconds after contact of the solvent with said fiber. It is more preferred that the shrinkage (process) starts between 4 and 120 seconds after contact of the solvent with said fiber. It is even more preferred that the shrinkage (process) starts between 20 and 60 seconds after contact of the solvent with said fiber. It is most preferred that the shrinkage (process) starts between 20 and 50 seconds after contact of the solvent with said fiber. For example, the shrinkage (process) starts between 3 and 200 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after contact of the solvent with said fiber. The temperature at the time of contact between the solvent and the fiber may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12.1, or 12.2.

It is preferred that the shrinkage (process) starts between 4 and 120 seconds after first contact with the solvent at a temperature of between 8 and 35°C, and/or at a pH of between pH 2.8 and 12.2. It is more preferred that the shrinkage (process) starts between 20 and 60 seconds after contact of the solvent with said fiber at a temperature of between 16 and 24°C, and/or at a pH of between pH 2.8 and 12.2. It is even more preferred that the shrinkage (process) starts between 4 and 25 seconds after contact of the solvent with said fiber at a temperature of between 24 and 35°C, and/or at a pH of between pH 2.8 and 12.2. It is most preferred that the shrinkage (process) starts between 25 and 45 seconds after contact of the solvent with said fiber at a temperature of between 16 and 24°C, and/or at a pH of between pH 6 and 8.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)). The temperature at the time of contact between the solvent and the fiber may be between 8°C and 37°C, e.g. between 8 and 35°C, 15 and 25°C, 16 and 24°C or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 550 seconds (after start of the shrinkage (process)) at a temperature of between 8 and 35°C, and/or at a pH of between pH 2.8 and 12.2. It is more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 110 and 275 seconds (after start of the shrinkage (process)) at a temperature of between 16 and 24°C, and/or at a pH of between pH 2.8 and 12.2. It is even more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 150 seconds (after start of the shrinkage (process)) at a temperature of between 24 and 35°C, and/or at a pH of between pH 2.8 and 12.2. It is most preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 115 and 230 seconds (after start of the shrinkage (process)) at a temperature of between 16 and 24°C, and/or at a pH of between pH 6 and 8.

Preferably, the shrinkage is irreversible. As mentioned above, irreversible means that the fiber does not return to its original shape without external impact/influences, in particular without force application, e.g. fiber extrusion, fiber extension, or fiber stretching. It is generally possible to re-extend the shrinked biopolymer fiber nearly to its original shape by techniques known to the person skilled in the art, e.g. by fiber extrusion, fiber extension, or fiber stretching. It is then possible to shrink the fiber by contacting said fiber with a solvent for a second time/again. However, the inventors of the present invention noticed that the shrinkage behavior of such a biopolymer fiber differs from a biopolymer fiber which shrinks after a first contact with a solvent.

Regarding the specific shrinkage behavior of the shrinkable biopolymer fiber which is influenceable/influenced by the following factors: temperature, pH, the thickness (diameter) of the biopolymer fiber, and/or the type of the biopolymer fiber used as a starting material (e.g. single-drawn or multi-drawn biopolymer fiber), it is referred to the first aspect of the present invention.

In a fourth aspect, the present invention relates to the use of a shrinkable biopolymer fiber for shaping an object.

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

Preferably, the object comprises or consists of a shrinkable biopolymer fiber. In one embodiment, the object comprises/consists of one single shrinkable biopolymer fiber. In another embodiment, the object comprises/consists of two or more shrinkable biopolymer fibers, e.g. a number of biopolymer fibers, or simply biopolymer fibers.

It is preferred that the object is a garment, apparel, a medical object, an orthopaedic object, a sports equipment including footwear, an outdoor equipment including footwear. It is also preferred that the object is a fabric, e.g. a woven fabric or knitted fabric. It is particularly preferred that the fabric, e.g. the woven fabric or knitted fabric, is a garment. The garment may be a fashion, a sport, an outdoor, a medical, or an orthopaedic garment. The garment may be fashion articles, a fashion goods, shirts, socks, stockings, e.g. compression stockings, medical stockings, or support stockings, tights, e.g. support tights, pants, e.g. sport or outdoor pants, underwear, e.g. sport or outdoor underwear, gloves, caps, storm hoods, footwear or bandages.

Particularly, the biopolymer fiber has a specific shrinkage behavior. Said specific shrinkage behavior is influenceable/influenced by temperature and/or pH. More particularly, the shrinkage (process) starts after a duration after first contact of the object with a solvent/after first contact of a solvent with the object and is concluded after a time range. As mentioned above, the object comprises or consists of a shrinkable biopolymer fiber. Thus, the shrinkage of said fiber finally results in the shaping of the object.

The inventors of the present patent application surprisingly found that an increase in temperature/a temperature increase reduces the duration between first contact of the solvent with said object and start of the shrinkage/shrinkage process ("time to shrink"), and/or that an increase in temperature/a temperature increase reduces the time range in which the shrinkage is concluded ("shrinkage duration"). In addition, the inventors of the present patent application surprisingly found that an increase in pH (towards a more basic pH) reduces the time range in which the shrinkage (process) is concluded.

It is preferred that the shrinkage (process) takes place at a temperature of between 8°C and 37°C, e.g. between 8 and 35°C, 15 and 25°C, 16 and 24°C, or 24 and 35°C. In other words, it is preferred that the temperature at the time of contacting the object with the solvent or the solvent with the object is between 8°C and 37°C, e.g. between 8 and 35°C, 15 and 25°C, 16 and 24°C, or 24 and 35°C. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C.

It is alternatively or additionally preferred that the shrinkage (process) takes place at a pH of between 2.8 and 12.2, e.g. between pH 6 and 8. In other words, it is preferred that the pH of the solvent is between pH 2.8 and 12.2, e.g. between pH 6 and 8. For example, the pH may be pH 2.8, 2.9, 3, 4, 5, 6, 7, 8, 9, 10, 11, 12, 12.1, or 12.2.

It is preferred that said fiber shows a shrinkage of at least 10% with regard to its total length after first contact of the object with a solvent. It is more preferred that said fiber shows a shrinkage of at least 15% with regard to its total length after first contact of the object with a solvent. It is even more preferred that said fiber shows a shrinkage of at least 25% with regard to its total length after first contact of the object with a solvent. It is most preferred that said fiber shows a shrinkage of at least 35% with regard to its total length after first contact of the object with a solvent. For example, said fiber shows a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to its total length after first contact of the object with a solvent. It is also preferred that said fiber shows a shrinkage of between 10% and 50% with regard to its total length after first contact of the object with a solvent. It is more preferred that said fiber shows a shrinkage of between 15% and 35% with regard to its total length after first contact of the object with a solvent. It is even more preferred that said fiber shows a shrinkage of between 15% and 25% with regard to its total length after first contact of the object with a solvent. For example, said fiber shows a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to its total length after first contact of the object with a solvent.

During shrinkage, the cross-sectional area of the biopolymer fiber increases.

It is further preferred that the shrinkage (process) starts between 3 and 200 seconds after first contact of the object with the solvent. It is more preferred that the shrinkage (process) starts between 4 and 120 seconds after first contact of the object with the solvent. It is even more preferred that the shrinkage (process) starts between 20 and 60 seconds after first contact of the object with the solvent. It is most preferred that the shrinkage (process) starts between 20 and 50 seconds after first contact of the object with the solvent. For example, the shrinkage (process) starts between 3 and 300 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after first contact of the object with the solvent. The temperature at the time of contacting the object with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage (process) starts between 4 and 120 seconds after first contact with the solvent at a temperature of between 8 and 35°C, and/or at a pH of between pH 2.8 and 12.2. It is more preferred that the shrinkage (process) starts between 20 and 60 seconds after first contact of the object with the solvent at a temperature of between 16 and 24°C, and/or at a pH of between pH 2.8 and 12.2. It is even more preferred that the shrinkage (process) starts between 4 and 25 seconds after first contact of the object with the solvent at a temperature of between 24 and 35°C, and/or at a pH of between pH 2.8 and 12.2. It is most preferred that the shrinkage (process) starts between 25 and 45 seconds after first contact of the object with the solvent at a temperature of between 16 and 24°C, and/or at a pH of between pH 6 and 8.

As the object comprises or consists of a shrinkable biopolymer fiber, the shrinkage of the biopolymer fiber results in the shaping of the object.

It should be clear to the skilled person that contacting the object with the solvent also results in contacting the shrinkable biopolymer fiber with the solvent as the object comprises or consists of the shrinkable biopolymer fiber.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)). The temperature at the time of contacting the shrinkable biopolymer fiber with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 550 seconds (after start of the shrinkage (process)) at a temperature of between 8 and 35°C, and/or at a pH of between pH 2.8 and 12.2. It is more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 110 and 275 seconds (after start of the shrinkage (process)) at a temperature of between 16 and 24°C, and/or at a pH of between pH 2.8 and 12.2. It is even more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 150 seconds (after start of the shrinkage (process)) at a temperature of between 24 and 35°C, and/or at a pH of between pH 2.8 and 12.2. It is most preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 115 and 230 seconds (after start of the shrinkage (process)) at a temperature of between 16 and 24°C, and/or at a pH of between pH 6 and 8.

Preferably, the shrinkage is irreversible. As mentioned above, irreversible means that the fiber does not return to its original shape without external impact/influences, in particular without force application, e.g. fiber extrusion, fiber extension, or fiber stretching. It is generally possible to re-extend the shrinked biopolymer fiber nearly to its original shape by techniques known to the person skilled in the art, e.g. by fiber extrusion, fiber extension, or fiber stretching. It is then possible to shrink the fiber by contacting said fiber with a solvent for a second time/again. However, the inventors of the present invention noticed that the shrinkage behavior of such a biopolymer fiber differs from a biopolymer fiber which shrinks after a first contact with a solvent.

The same applies to the object which comprises or consists of the fiber.

The solvent may be an aqueous solution or a solution comprising alcohol. It is preferred that the aqueous solution is a buffered aqueous solution, such as Tris/HCl, or water (H₂O), such as technical H₂O or deionized H₂O. It is also preferred that the alcohol is ethanol or isopropanol.

It is preferred that the biopolymer is a silk polypeptide. It is more preferred that the biopolymer is a recombinant silk polypeptide as already described under the first aspect.

It is further particularly preferred that the silk polypeptide comprises at least one non-repetitive (NR) unit as already described under the first aspect.. In one preferred embodiment, the silk polypeptide is selected from the group consisting of (C)ₘ, (C^{Cys})ₘ, (C^{kappa})ₘ, (C)ₘC^{Cys}, C^{Cys}(C)ₘ, (C)ₘC^{Cys}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, C^{kappa}₁₆, C^{kappa}₃₂, C^{kappa}48, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C_{48.}

As mentioned above, the biopolymer fiber has a specific shrinkage behavior. Said specific shrinkage behavior is further influenceable/influenced by the thickness (diameter) of the biopolymer fiber. More particularly, the shrinkage (process) starts after a duration after first contact of the object with a solvent/after first contact of a solvent with the object and is concluded after a time range. As mentioned above, the object comprises or consists of a shrinkable biopolymer fiber. Thus, the shrinkage of said fiber finally results in the shaping of the object. The inventors of the present patent application surprisingly found that an increase in the thickness (diameter) of the biopolymer fiber increases the duration between first contact of the object with a solvent/after first contact of a solvent with the object and start of the shrinkage (process), and/or that an increase in the thickness (diameter) of the biopolymer fiber increases the time range in which the shrinkage is concluded. Preferably, the biopolymer fiber has a thickness (diameter) of between 5 µm and 200 µm. More preferably, the biopolymer fiber has a thickens (diameter) of between 50 µm and 150 µm.

Considering the aforementioned, the biopolymer fiber has a specific shrinkage behavior which is influenceable/influenced by the following factors: temperature, pH, and/or the diameter of the biopolymer fiber.

Moreover, the inventors of the present patent application surprisingly found that the extent of shrinkage with regard to the total length of the biopolymer fiber can be influenced by the type of biopolymer fiber used as a starting material. In particular, they surprisingly found that the extent of shrinkage with regard to the total length of the biopolymer fiber is influenceable/influenced by the degree of drawing of the biopolymer fiber used as a starting material. As mentioned above, the biopolymer fiber may be a single-drawn or a multi-drawn (e.g. double-drawn) fiber. Said fiber has been stretched one or more times during its preparation process, in particular wet-spinning process. The inventors of the present patent application now observed that the extend of shrinkage with regard to the total length of the biopolymer fiber is higher, the higher the degree of drawing of the biopolymer fiber used as a starting material is. For example, the extend of shrinkage is higher with regard to the total length of the biopolymer fiber with a multi-drawn (e.g. double-drawn) biopolymer fiber (e.g. at least 20%) than with a single-drawn biopolymer fiber (e.g. at least 10%).

Based on the above, the biopolymer fiber has a specific shrinkage behavior which is influenceable/influenced by the following factors: temperature, pH, the diameter of the biopolymer fiber, and/or the type of the biopolymer fiber used as a starting material (e.g. single-drawn or multi-drawn biopolymer fiber).

The fourth aspect of the present invention, as described above, can alternatively be worded as follows: In a fourth aspect, the present invention relates to a method for shaping an object, wherein a shrinkable biopolymer fiber is used.

In a fifth aspect, the present invention relates to a method for shaping an object comprising the steps of:
(i) providing an object comprising or consisting of a shrinkable biopolymer fiber, and
(ii) (first) contacting said object with a solvent, thereby shaping the object.

In step (i) of the method of the fifth aspect of the present invention, an object comprising or consisting of shrinkable biopolymer fibers is provided. It is preferred that the biopolymer is a silk polypeptide. It is more preferred that the biopolymer is a recombinant silk polypeptide as already described under the first aspect.

It is further particularly preferred that the silk polypeptide comprises at least one non-repetitive (NR) unit as already described under the first aspect.. Said non-repetitive (NR) unit may be comprised at the N- and/or C-terminus.

In one preferred embodiment, the silk polypeptide is selected from the group consisting of (C)ₘ, (C^{Cys})m, (C^{kappa})ₘ, (C)ₘC^{Cys}, C^{Cys}(C)ₘ, (C)ₘC^{Cys}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, C^{kappa}₁₆, C^{kappa}₃₂, C^{kappa}48, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C_{48.}

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

As mentioned in step (i) of the method of the fifth aspect of the present invention, the object comprises or consists of a shrinkable biopolymer fiber. In one embodiment, the object comprises/consists of one single shrinkable biopolymer fiber. In another embodiment, the object comprises/consists of two or more shrinkable biopolymer fibers, e.g. a number of biopolymer fibers.

It is preferred that the object is a garment, apparel, a medical object, an orthopaedic object, a sports equipment including footwear, an outdoor equipment including footwear. It is also preferred that the object is a fabric, e.g. a woven fabric or knitted fabric. It is particularly preferred that the fabric, e.g. the woven fabric or knitted fabric, is a garment. The garment may be a fashion, a sport, an outdoor, a medical, or an orthopaedic garment. The garment may be fashion articles, fashion goods, shirts, socks, stockings, e.g. compression stockings, medical stockings, or support stockings, tights, e.g. support tights, pants, e.g. sport or outdoor pants, underwear, e.g. sport or outdoor underwear, gloves, caps, storm hoods, footwear or bandages.

The object which is provided in step (i) of the method of the fifth aspect of the present invention may be mounted on a molded article, e.g. on an artificial body part. The body part may also be from a living human being, e.g. a human hand in case that the object which is to be shaped is a glove or a human leg/foot in case that the object which is to be shaped is a bandage/footwear.

The method of the fifth aspect of the present invention, thus, allows the manufacture of tailored and/or customized objects.

In step (ii) of the method of the fifth aspect of the present invention, said object is contacted with a solvent. The contact of the object with the solvent may occur by dropping the solvent onto said object, by dipping said object into the solvent, or by spraying the solvent onto said object. It should be clear to the skilled person that contacting the object with the solvent also results in contacting the shrinkable biopolymer fiber with the solvent as the object comprises or consists of the shrinkable biopolymer fiber.

The solvent may be an aqueous solution or a solution comprising alcohol. It is preferred that the aqueous solution is a buffered aqueous solution, such as Tris/HCl, or water (H₂O), such as technical H₂O or deionized H₂O. It is also preferred that the alcohol is ethanol or isopropanol.

As mentioned above, the object comprises or consists of a shrinkable biopolymer fiber. After (first) contact of the object with the solvent, the biopolymer fiber comprised therein starts shrinking. This results in the shaping of the object. Preferably the biopolymer fiber shows a shrinkage of at least 10%, preferably of at least 15%, more preferably of at least 25%, even more preferably of at least 35%, with regard to its total length after (first) contact of said object with the solvent. For example, the biopolymer fiber show a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to its total length after (first) contact of said object with the solvent. It is also preferred that said fiber shows a shrinkage of between 10% and 50% with regard to its total length after (first) contact of said object with the solvent. It is more preferred that said fiber shows a shrinkage of between 15% and 35% with regard to its total length after (first) contact of said object with the solvent. It is even more preferred that said fiber shows a shrinkage of between 15% and 25% with regard to its total length after (first) contact of said object with the solvent. For example, said fiber shows a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to its total length after first contact of said object with the solvent.

It is further preferred that the shrinkage (process) starts between 3 and 200 seconds after (first) contact of the object with the solvent. It is more preferred that the shrinkage (process) starts between 4 and 120 seconds after (first) contact of the object with the solvent. It is even more preferred that the shrinkage (process) starts between 20 and 60 seconds after (first) contact of the object with the solvent. It is most preferred that the shrinkage (process) starts between 20 and 50 seconds after (first) contact of the object with the solvent. For example, the shrinkage (process) starts between 3 and 200 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after (first) contact of the object with the solvent. The temperature at the time of contacting the object with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage (process) starts between 4 and 120 seconds after first contact with the solvent at a temperature of between 8 and 35°C, and/or at a pH of between pH 2.8 and 12.2. It is more preferred that the shrinkage (process) starts between 20 and 60 seconds after (first) contact of the object with the solvent at a temperature of between 16 and 24°C, and/or at a pH of between pH 2.8 and 12.2. It is even more preferred that the shrinkage (process) starts between 4 and 25 seconds after (first) contact of the object with the solvent at a temperature of between 24 and 35 °C, and/or at a pH of between pH 2.8 and 12.2. It is most preferred that the shrinkage (process) starts between 25and 45 seconds after (first) contact of the object with the solvent at a temperature of between 16 and 24°C, and/or at a pH of between pH 6 and 8.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, more preferably of between 110 and 250 seconds (after start of the shrinkage (process)). It is even more preferred that the shrinkage is concluded to 100% after a time range of between 30 and 700 seconds, preferably of between 33 and 550 seconds, more preferably of between 110 and 250 seconds (after start of the shrinkage (process)). The temperature at the time of contacting the object with the solvent may be between 8 and 37°C, e.g. between 8 and 35°C, 15 and 25°C, 16 and 24°C, or 24 and 35°C, and/or the pH of the solvent may be between pH 2.8 and 12.2, e.g. between pH 6 and 8. For example, the temperature may be 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37°C and/or the pH may be pH 2.8, 2.9, 3, 4, 5, 6, 7, 8, 9, 10, 11, 12, 12.1, or 12.2.

It is preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 550 seconds (after start of the shrinkage (process)) at a temperature of between 8 and 35°C, and/or at a pH of between pH 2.8 and 12.2. It is more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 110 and 275 seconds (after start of the shrinkage (process)) at a temperature of between 16 and 24°C, and/or at a pH of between pH 2.8 and 12.2. It is even more preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 33 and 150 seconds (after start of the shrinkage (process)) at a temperature of between 24 and 35°C, and/or at a pH of between pH 2.8 and 12.2. It is most preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 115and 230 seconds (after start of the shrinkage (process)) at a temperature of between 16 and 24°C, and/or at a pH of between pH 6 and 8.

Preferably, the shrinkage is irreversible. As mentioned above, irreversible means that the fiber does not return to its original shape without external impact/influences, in particular without force application, e.g. fiber extrusion, fiber extension, or fiber stretching. It is generally possible to re-extend the shrinked biopolymer fiber nearly to its original shape by techniques known to the person skilled in the art, e.g. by fiber extrusion, fiber extension, or fiber stretching. It is then possible to shrink the fiber by contacting said fiber with a solvent for a second time/again. However, the inventors of the present invention noticed that the shrinkage behavior of such a biopolymer fiber differs from a biopolymer fiber which shrinks after a first contact with a solvent.

The same applies to the object which comprises or consist of shrinkable biopolymer fibers.

It will be understood by the skilled person that the shrinkage in a single biopolymer fiber takes place one-dimensional. In contrast thereto, the shrinkage in an object comprising or consisting of biopolymer fibers may take place multidimensional. For example, the shrinkage in a woven cloth comprising or consisting of biopolymer fibers which are weaved to each other takes place multidimensional.

The method of the fifth aspect of the present invention further comprises the step of drying the object. Drying the object may be achieved by any process known to the skilled person. The drying can be carried out, for example, by drying in the air, baking, using a heat chamber, a vacuum chamber, laminar flow (e.g. of a gas such as nitrogen or carbon dioxide), radiation, or a fan (at low temperatures, at room temperature or at elevated temperatures).

Alternatively or additionally, the method of the fifth aspect of the present invention further comprises the step of fixing the object. If the object is mounted on a molded article, it may be removed from said article after fixation. Fixing the object has the effect that the formed object does not change its form/shape anymore, in particular the form/shape the object has taken on the molded article. The object can be fixed by chemical or physical means. For fixing the object, one or more reagents selected from the group consisting of glues and resins such as epoxy resins or polyester resins may be used. The fixation of the object may occur by dropping the one or more fixation reagents onto said object, by dipping said object into the one or more fixation reagents by spraying the one or more fixation reagents onto said object, by painting said object with the one or more fixation reagents, or by applying the one or more fixation reagents onto said object with a brush. Alternatively the object can be fixed mechanically e.g. by knotting or clamping.

As mentioned above, the object may also be mounted on a body part from a living human being, e.g. a human leg/foot in case that the object which is to be shaped is a bandage, a stocking or a footwear. In both cases, the shrinkage of the biopolymer fiber results in the compression of the body part from a living human being, e.g. the human leg. This may be desired in the field of sport, e.g. to guarantee sufficient blood flow to the heart.

In a sixth aspect, the present invention relates to the use of a shrinkable biopolymer fiber as suture material. The shrinkable biopolymer fiber can be applied as suture material to wounds. The suture material is preferably a suture, e.g. a surgical suture. The shrinkable biopolymer fiber may be part of a thread. The thread comprises or consists of shrinkable biopolymer fibers.

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

Particularly, the biopolymer fiber has a specific shrinkage behavior. More particularly, the shrinkage (process) starts after a duration after first contact of the biopolymer fiber with a liquid (as solvent), e.g. a liquid present at the place of a wound such as tissue fluid, blood, or sore fluid/after first contact of a liquid, e.g. a liquid present at the place of a wound such as tissue fluid, blood, or score fluid with the biopolymer fiber and is concluded after a time range. Thus, the contact of the biopolymer fiber as suture with a tissue fluid, blood, or score fluid (tissue fluid, blood as well as score fluid are aqueous solutions containing water as solvent) at the place of a wound induces shrinkage of the biopolymer fiber which results in tightening the margins of the wound after suturing. The use of the shrinkable biopolymer fiber as suture preferably allows the treatment of cuts or gaping wounds.

Preferably, the shrinkage (process) takes place at a temperature of between 36°C and 40°C, more preferably at 37°C.

It is preferred that said fiber shows a shrinkage of at least 10% with regard to its total length after first contact with a liquid. It is more preferred that said fiber shows a shrinkage of at least 15% with regard to its total length after first contact with a liquid. It is even more preferred that said fiber shows a shrinkage of at least 25% with regard to its total length after first contact with a liquid. It is most preferred that said fiber shows a shrinkage of at least 35% with regard to its total length after first contact with a liquid. For example, said fiber shows a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to its total length after first contact with a liquid. It is also preferred that said fiber shows a shrinkage of between 10% and 50% with regard to its total length after first contact with a liquid. It is more preferred that said fiber shows a shrinkage of between 15% and 35% with regard to its total length after first contact with a liquid. It is even more preferred that said fiber shows a shrinkage of between 15% and 25% with regard to its total length after first contact with a liquid. For example, said fiber shows a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to its total length after first contact with a liquid.

During shrinkage, the cross-sectional area of the biopolymer fiber increases.

It is further preferred that the shrinkage (process) starts between 3 and 200 seconds after first contact with the liquid. It is more preferred that the shrinkage (process) starts between 4 and 120 seconds after first contact with the liquid. It is even more preferred that the shrinkage (process) starts between 20 and 60 seconds after first contact with the liquid. It is most preferred that the shrinkage (process) starts between 20 and 50 seconds after first contact with the liquid. For example, the shrinkage (process) starts between 3 and 300 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after first contact with the liquid.

The liquid is preferably a liquid present at the place of a wound, e.g. a tissue fluid, blood, or sore fluid.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)).

If the moisture content or rest moisture content at the place of the wound is not sufficient for biopolymer fiber shrinkage, the biopolymer fiber may additionally or alternatively be wetted or moistened with another solvent, e.g. water or saline solution such as isotonic saline solution.

Preferably, the shrinkage is irreversible. As mentioned above, irreversible means that the fiber does not return to its original shape without external impact/influences, in particular without force application, e.g. fiber extrusion, fiber extension, or fiber stretching.

Preferably, the fiber is coated/finished.

It is preferred that the biopolymer is a silk polypeptide. It is more preferred that the biopolymer is a recombinant silk polypeptide as already described under the first aspect.

It is further particularly preferred that the silk polypeptide comprises at least one non-repetitive (NR) unit as already described under the first aspect.. In one preferred embodiment, the silk polypeptide is selected from the group consisting of (C)ₘ, (C^{Cys})ₘ, (C^{kappa})ₘ, (C)ₘC^{Cys}, C^{Cys}(C)ₘ, (C)ₘC^{Cys}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, C^{kappa}₁₆, C^{kappa}₃₂, C^{kappa}48, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C_{48.}

Preferably, the biopolymer fiber has a thickness (diameter) of between 5 µm and 200 µm. More preferably, the biopolymer fiber has a thickens (diameter) of between 50 µm and 150 µm.

The inventors of the present patent application surprisingly found that the extent of shrinkage with regard to the total length of the biopolymer fiber can be influenced by the type of biopolymer fiber used as a starting material. For example, the extend of shrinkage is higher with regard to the total length of the biopolymer fiber with a multi-drawn (e.g. double-drawn) biopolymer fiber (e.g. at least 20%) than with a single-drawn biopolymer fiber (e.g. at least 10%).

The sixth aspect of the present invention, as described above, can alternatively be worded as follows: In a sixth aspect, the present invention relates to a method for suturing a wound, wherein a shrinkable biopolymer fiber is used (as suture material). In particular, the shrinkable biopolymer fiber is applied to wounds as suture material. By suturing a wound with the shrinkable biopolymer fiber, said fiber comes into contact with liquid at the place of the wound, e.g. tissue fluid, blood, or sore fluid. The contact of the biopolymer fiber as suture with the liquid induces shrinkage of the biopolymer fiber which results in tightening the margins of the wound after suturing. This preferably allows the treatment of cuts or gaping wounds of a subject. If the moisture content or rest moisture content at the place of the wound is not sufficient for biopolymer fiber shrinkage, the biopolymer fiber may additionally or alternatively be wetted or moistened with another solvent, e.g. water or saline solution such as isotonic saline solution. The sixth aspect of the present invention, as described above, can also alternatively be worded as follows: In a sixth aspect, the present invention relates to a shrinkable biopolymer fiber for use as a suture material.

In a seventh aspect, the present invention relates to the use of a shrinkable biopolymer fiber as wound dressing.

In particular, the shrinkable biopolymer fiber is applied to wounds as wound dressing. The wound dressing preferably comprises or consists of the shrinkable biopolymer fiber. It is preferred that the wound dressing has the form of a tissue, woven fabric or non-woven fabric. It is also preferred that the shrinkable biopolymer fiber is part of a tissue, woven fabric or non-woven fabric. In other words, the tissue, woven fabric or non-woven fabric preferably comprises or consists of the shrinkable biopolymer fiber which is used as wound dressing. Techniques to produce a tissue, woven fabric or non-woven fabric out of a shrinkable biopolymer fiber are known to the skilled person. It is alternatively preferred that the shrinkable biopolymer is part of a composite material (e.g. a plaster or bandage).

It is preferred that the biopolymer fiber has a residual moisture content of no more than 20%, e.g. no more than 20, 19, 18, 17, 16, 15, 14, 13, 12, 11, 10, 9, 8, 7, 6, 5, 4, 3, 2, 1%, or of 0%. It is more preferred that the biopolymer fiber has a residual moisture content of no more than 10%, e.g. no more than 10, 9, 8, 7, 6, 5, 4, 3, 2, 1%, or of 0%. In case of a residual moisture content of 0%, the fiber is dry. Preferably the moisture content of the fiber is between 2 % and 10 %.

It is further (alternatively or additionally) preferred that the biopolymer fiber has a linear density of 1-700 Decitex (dtex). It is more preferred that the biopolymer fiber has a linear density of 10-300 dtex. It is even more preferred that the biopolymer fiber has a linear density of 50-250 dtex. It is most preferred that the biopolymer fiber has a linear density of 60-200 dtex.

It is also (alternatively or additionally) preferred that the biopolymer fiber has a thickness (diameter) of between 0.5 µm and 300 µm. It is more preferred that the biopolymer fiber has a thickness (diameter) of between 1 µm and 200 µm.

Particularly, the biopolymer fiber has a specific shrinkage behavior. More particularly, the shrinkage (process) starts after a duration after first contact of the biopolymer fiber with a liquid (as solvent), e.g. a liquid present at the place of a wound such as tissue fluid, blood, or sore fluid/after first contact of a liquid, e.g. a liquid present at the place of a wound such as tissue fluid, blood, or score fluid with the biopolymer fiber and is concluded after a time range. Thus, the contact of the biopolymer fiber as wound dressing with a tissue fluid, blood, or score fluid (tissue fluid, blood as well as score fluid are aqueous solutions containing water as solvent) at the place of a wound induces shrinkage of the biopolymer fiber as component of the wound dressing or composite material which results in tightening the margins of the wound or compression of the wound.

Preferably, the shrinkage (process) takes place at a temperature of between 36°C and 40°C, more preferably at 37°C.

It is preferred that said fiber shows a shrinkage of at least 10% with regard to its total length after first contact with a liquid. It is more preferred that said fiber shows a shrinkage of at least 15% with regard to its total length after first contact with a liquid. It is even more preferred that said fiber shows a shrinkage of at least 25% with regard to its total length after first contact with a liquid. It is most preferred that said fiber shows a shrinkage of at least 35% with regard to its total length after first contact with a liquid. For example, said fiber shows a shrinkage of at least 10, 11, 12, 13, 14, 15, 16, 17, 18, 19, 20, 21, 22, 23, 24, 25, 26, 27, 28, 29, 30, 31, 32, 33, 34, or 35% with regard to its total length after first contact with a liquid. It is also preferred that said fiber shows a shrinkage of between 10% and 50% with regard to its total length after first contact with a liquid. It is more preferred that said fiber shows a shrinkage of between 15% and 35% with regard to its total length after first contact with a liquid. It is even more preferred that said fiber shows a shrinkage of between 15% and 25% with regard to its total length after first contact with a liquid. For example, said fiber shows a shrinkage of between 10% and 50%, between 11% and 49%, between 12% and 48%, between 13% and 47%, between 14% and 46%, between 15% and 45%, between 16% and 44%, between 17% and 43%, between 18% and 42%, between 19% and 41%, between 20% and 40%, between 21% and 39%, between 22% and 38%, between 23% and 37%, between 24% and 36%, between 25% and 35%, between 26% and 34%, between 27% and 33%, between 28% and 32%, and between 29% and 31% with regard to its total length after first contact with a liquid.

During shrinkage, the cross-sectional area of the biopolymer fiber increases.

It is further preferred that the shrinkage (process) starts between 3 and 200 seconds after first contact with the liquid. It is more preferred that the shrinkage (process) starts between 4 and 120 seconds after first contact with the liquid. It is even more preferred that the shrinkage (process) starts between 20 and 60 seconds after first contact with the liquid. It is most preferred that the shrinkage (process) starts between 20 and 50 seconds after first contact with the liquid. For example, the shrinkage (process) starts between 3 and 300 seconds, between 4 and 150 seconds, between 5 and 140 seconds, between 6 and 130 seconds, between 7 and 120 seconds, between 8 and 110 seconds, between 9 and 100 seconds, between 10 and 60 seconds, between 11 and 58 seconds, between 12 and 55 seconds, between 13 and 52 seconds, between 14 and 50 seconds, between 15 and 49 seconds, between 16 and 48 seconds, or between 17 and 47 seconds after first contact with the liquid.

The liquid is preferably a liquid present at the place of a wound, e.g. a tissue fluid, blood, or sore fluid.

It is also preferred that the shrinkage is concluded to at least 80%, e.g. at least 80, 81, 82, 83, 84, 85, 86, 87, 88, 89, 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is more preferred that the shrinkage is concluded to at least 90%, e.g. at least 90, 91, 92, 93, 94, 95, 96, 97, 98, 99%, or 100%, after a time range of between 30 and 700 seconds, preferably of between 33 and 550 seconds, and more preferably of between 110 and 250 seconds (after start of the shrinkage (process)). It is even more preferred that the shrinkage is concluded to 100% after a time range of between 30 and 700 seconds, preferably of between 33 and 550 seconds, and more preferably of between 110 and 250 seconds (after start of the shrinkage (process)).

If the moisture content or rest moisture content at the place of the wound is not sufficient for biopolymer fiber shrinkage, the biopolymer fiber may additionally or alternatively be wetted or moistened with another solvent, e.g. water or saline solution such as isotonic saline solution.

Preferably, the shrinkage is irreversible. As mentioned above, irreversible means that the fiber does not return to its original shape without external impact/influences, in particular without force application, e.g. fiber extrusion, fiber extension, or fiber stretching.

It is preferred that the biopolymer is a silk polypeptide. It is more preferred that the biopolymer is a recombinant silk polypeptide as already described under the first aspect.

It is further particularly preferred that the silk polypeptide comprises at least one non-repetitive (NR) unit as already described under the first aspect.. In one preferred embodiment, the silk polypeptide is selected from the group consisting of (C)ₘ, (C^{Cys})ₘ, (C^{kappa})ₘ, (C)ₘC^{Cys}, C^{Cys}(C)ₘ, (C)ₘC^{Cys}(C)ₘ, (C)ₘNR_{z}, NR_{z}(C)ₘ and NR_{z}(C)ₘNR_{z}, , wherein m is an integer of 8 to 96, i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61,62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 or 96, z is an integer of 1 to 3, i.e. 1, 2, or 3, and NR stands for a non-repetitive unit.

In one more preferred embodiment, the silk polypeptide is selected from the group consisting of C₈, C₁₆, C₃₂, C₄₈, C^{kappa}₈, C^{kappa}₁₆, C^{kappa}₃₂, C^{kappa}₄₈, C₈C^{Cys}, C₁₆C^{Cys}, C₃₂C^{Cys}, C₄₈C^{Cys}, C^{Cys}C₈, C^{Cys}C₁₆, C^{Cys}C₃₂, and C^{Cys}C₄₈.

Preferably, the biopolymer fiber has a thickness (diameter) of between 5 µm and 200 µm. More preferably, the biopolymer fiber has a thickens (diameter) of between 50 µm and 150 µm.

The inventors of the present patent application surprisingly found that the extent of shrinkage with regard to the total length of the biopolymer fiber can be influenced by the type of biopolymer fiber used as a starting material. For example, the extend of shrinkage is higher with regard to the total length of the biopolymer fiber with a multi-drawn (e.g. double-drawn) biopolymer fiber (e.g. at least 20%) than with a single-drawn biopolymer fiber (e.g. at least 10%).

The seventh aspect of the present invention, as described above, can alternatively be worded as follows: In a seventh aspect, the present invention relates to a method for treating/covering a wound with a shrinkable biopolymer fiber (as wound dressing). In particular, the shrinkable biopolymer fiber is applied to wounds as wound dressing. The wound dressing preferably comprises or consists of the shrinkable biopolymer fiber. It is preferred that the wound dressing has the form of a tissue, woven fabric or non-woven fabric. It is also preferred that the shrinkable biopolymer fiber is part of a tissue, woven fabric or non-woven fabric. In other words, the tissue, woven fabric or non-woven fabric preferably comprises or consists of the shrinkable biopolymer fiber which is used as wound dressing. It is alternatively preferred that the shrinkable biopolymer is part of a composite material (e.g. a plaster or bandage).

By treating/covering a wound with the shrinkable biopolymer fiber, said fiber comes into contact with liquid at the place of the wound, e.g. tissue fluid, blood, or sore fluid. The contact of the biopolymer fiber as wound dressing with the liquid induces shrinkage of the biopolymer fiber which results in tightening the margins of the wound or compression of the wound. If the moisture content or rest moisture content at the place of the wound is not sufficient for biopolymer fiber shrinkage, the biopolymer fiber may additionally or alternatively be wetted or moistened with another solvent, e.g. water or saline solution such as isotonic saline solution. The seventh aspect of the present invention, as described above, can also alternatively be worded as follows: In a seventh aspect, the present invention relates to a shrinkable biopolymer fiber for use as wound dressing.

It should be noted that the uses and methods described herein are preferably non-therapeutic uses and/or methods.

The present invention is summarized as follows:
1. Use of a shrinkable biopolymer fiber as sensor.
2. The use of the shrinkable biopolymer fiber of item 1, wherein said fiber has a specific shrinkage behavior which is influenceable by temperature and/or pH.
3. The use of the shrinkable biopolymer fiber of items 1 or 2, wherein said fiber shows a shrinkage of at least 10% with regard to its total length after first contact with a solvent.
4. The use of the shrinkable biopolymer fiber of items 1 to 3, wherein the sensor allows to determine the authenticity of a product.
5. The use of the shrinkable biopolymer fiber of items 2 to 4, wherein the shrinkage is indicative for the authenticity of a product.
6. The use of the shrinkable biopolymer fiber of any one of items 1 to 5, wherein the sensor allows to determine the presence of a solvent.
7. The use of the shrinkable biopolymer fiber of any one of items 2 to 6, wherein the shrinkage is indicative for the presence of a solvent.
8. A method for determining the authenticity of a product comprising the steps of:
   (i) providing a shrinkable biopolymer fiber as sensor,
   (ii) contacting said fiber with a solvent, and
   (iii) observing whether a shrinkage of said fiber after contact with the solvent occurs/occurred,
      wherein a shrinkage of at least 10% with regard to the total length of said fiber is indicative for the authenticity of the product.
9. A method for determining the presence of a solvent comprising the steps of:
   (i) providing a shrinkable biopolymer fiber as sensor, and
   (ii) observing whether a shrinkage of said fiber occurs/occurred,
      wherein a shrinkage of at least 10% with regard to the total length of said fiber is indicative for the presence of a solvent in contact with said fiber.
10. The use of the shrinkable biopolymer fiber of any one of items 3 to 7, or the method of items 8 or 9, wherein the shrinkage (process) starts between 3 and 200 seconds after (first) contact with the solvent/after (first) contact of the solvent with said fiber.
11. The use of the shrinkable biopolymer fiber or the method of item 10, wherein the shrinkage (process) starts between 4 and 120 seconds, preferably between 20 and 60 seconds, and more preferably between 20 and 50 seconds, after (first) contact with the solvent/after (first) contact of the solvent with said fiber.
12. The use of the shrinkable biopolymer fiber of any one of items 3 to 7, 10 or 11, or the method of any one of items 8 to 11, wherein said fiber shows a shrinkage of between 10% and 50%, preferably of between 15% and 35%, and more preferably of between 15% and 25%.
13. The use of the shrinkable biopolymer fiber of any one of items 3 to 7 or 10 to 12, or the method of any one of items 8 to 12, wherein the shrinkage is concluded to at least 80% after a time range of between 30 and 700 seconds, preferably of between 33 and 550 seconds, and more preferably of between 110 and 250 seconds (after start of the shrinkage (process)).
14. The use of the shrinkable biopolymer fiber of any one of items 2 to 7 or 10 to 13, or the method of any one of items 8 to 13, wherein the shrinkage is irreversible.
15. The use of the shrinkable biopolymer fiber of any one of items 3 to 7 or 10 to 14, or the method of any one of items 8 to 14, wherein the solvent is an aqueous solution, preferably water, or a solution comprising alcohol.
16. The use of the shrinkable biopolymer fiber of any one of items 1 to 7 or 10 to 15, or the method of any one of items 8 to 15, wherein the biopolymer is a silk polypeptide.
17. The use of the shrinkable biopolymer fiber or the method of item 16, wherein the silk polypeptide is a recombinant silk polypeptide.
18. The use of the shrinkable biopolymer fiber or the method of items 16 or 17, wherein the silk polypeptide comprises at least two identical repetitive units.
19. The use of the shrinkable biopolymer fiber or the method of item 18, wherein the repetitive units are independently selected from the group consisting of module C having the sequence according to SEQ ID NO: 1 or a variant thereof, module C^{Cys} having the sequence according to SEQ ID NO: 2, and module C^{kappa} having the sequence according to SEQ ID NO: 3.
20. The use of the shrinkable biopolymer fiber or the method of any one of items 16 to 19, wherein the silk polypeptide comprises at least one non-repetitive (NR) unit.
21. The use of the shrinkable biopolymer fiber of any one of items 4 to 7 or 10 to 20, or the method of any one of items 8 or 10 to 20, wherein the product is a fabric.
22. The use of the shrinkable biopolymer fiber or the method of item 21, wherein the product is a woven fabric or knitted fabric.
23. The use of the shrinkable biopolymer fiber or the method of items 21 or 22, wherein the fabric is an apparel or a garment.
24. The use of the shrinkable biopolymer fiber of any one of items 1 to 7 or 10 to 23, or the method of any one of items 8 or 10 to 23, wherein said fiber is part of a label or hangtag.
25. The use of the shrinkable biopolymer fiber or the method of item 24, wherein the label or hangtag is attached to the product or is part of the product, or wherein the label or hangtag is attached to the packaging or is part of the packaging comprising the product.
26. Use of a shrinkable biopolymer fiber for shaping an object.
27. The use of the shrinkable biopolymer fiber of item 26, wherein said fiber has a specific shrinkage behavior which is influenceable by temperature and/or pH.
28. The use of the shrinkable biopolymer fiber of items 26 or 27, wherein the object comprises or consists of a shrinkable biopolymer fiber.
29. The use of the shrinkable biopolymer fiber of items 26 to 28, wherein said fiber shows a shrinkage of at least 10% with regard to its total length after first contact of the object with a solvent.
30. The use of the shrinkable biopolymer fiber of item 29, wherein the shrinkage of said fiber results in the shaping of the object.
31. A method for shaping an object comprising the steps of:
   (i) providing an object comprising or consisting of a shrinkable biopolymer fiber,
   (ii) contacting said object with a solvent, thereby shaping the object.
32. The method of item 31, wherein said fiber shows a shrinkage of at least 10% with regard to its total length after first contact of said object with the solvent.
33. The use of the shrinkable biopolymer fiber of items 29 or 30, or the method of item 32, wherein the shrinkage (process) starts between 3 and 200 seconds after (first) contact of said object with the solvent.
34. The use of the shrinkable biopolymer fiber or the method of item 33, wherein the shrinkage (process) starts between 4 and 120 seconds, preferably between 20 and 60 seconds, and more preferably between 20 and 50 seconds, after (first) contact of said object with the solvent.
35. The use of the shrinkable biopolymer fiber of items 29, 30, 33 or 34, or the method of any one of items 32 to 34, wherein said fiber shows a shrinkage of between 10% and 50%, preferably of between 15% and 35%, and more preferably of between 15% and 25%.
36. The use of the shrinkable biopolymer fiber of any one of items 29, 30, 33 to 35, or the method of any one of items 32 to 35, wherein the shrinkage is concluded to at least 80% after a time range of between 30 and 700 seconds, preferably of between 33 and 550 seconds, and more preferably of between 110 and 250 seconds (after start of the shrinkage (process)).
37. The use of the shrinkable biopolymer fiber of any one of items 29, 30, 33 to 36, or the method of any one of items 32 to 36, wherein the shrinkage is irreversible.
38. The use of the shrinkable biopolymer fiber of any one of items 29, 30 or 33 to 37, or the method of any one of items 31 to 37, wherein the solvent is an aqueous solution, preferably water, or a solution comprising alcohol, preferably ethanol or isopropanol.
39. The use of the shrinkable biopolymer fiber of any one of items 26 to 30 or 33 to 38, or the method of any one of items 31 to 38, wherein the biopolymer is a silk polypeptide.
40. The use of the shrinkable biopolymer fiber or the method of item 39, wherein the silk polypeptide is a recombinant silk polypeptide.
41. The use of the shrinkable biopolymer fiber or the method of items 39 or 40, wherein the silk polypeptide comprises at least two identical repetitive units.
42. The use of the shrinkable biopolymer fiber or the method of item 41, wherein the repetitive units are independently selected from the group consisting of module C having the sequence according to SEQ ID NO: 1 or a variant thereof, module C^{Cys} having the sequence according to SEQ ID NO: 2, and module C^{kappa} having the sequence according to SEQ ID NO: 3.
43. The use of the shrinkable biopolymer fiber or the method of any one of items 39 to 42, wherein the silk polypeptide comprises at least one non-repetitive (NR) unit.

The present invention is further summarized as follows:
1. Use of a shrinkable biopolymer fiber as sensor.
2. The use of the shrinkable biopolymer fiber of item 1, wherein said fiber has a specific shrinkage behavior which is influenceable by temperature and/or pH.
3. The use of the shrinkable biopolymer fiber of items 1 or 2, wherein said fiber shows a shrinkage of at least 10% with regard to its total length after first contact with a solvent.
4. The use of the shrinkable biopolymer fiber of items 1 to 3, wherein the sensor allows to determine the authenticity of a product.
5. The use of the shrinkable biopolymer fiber of items 2 to 4, wherein the shrinkage is indicative for the authenticity of a product.
6. The use of the shrinkable biopolymer fiber of any one of items 1 to 5, wherein the sensor allows to determine the presence of a solvent.
7. The use of the shrinkable biopolymer fiber of any one of items 2 to 6, wherein the shrinkage is indicative for the presence of a solvent.
8. A method for determining the authenticity of a product comprising the steps of:
   (i) providing a shrinkable biopolymer fiber as sensor,
   (ii) contacting said fiber with a solvent, and
   (iii) observing whether a shrinkage of said fiber after contact with the solvent occurs, wherein a shrinkage of at least 10% with regard to the total length of said fiber is indicative for the authenticity of the product.
9. A method for determining the presence of a solvent comprising the steps of:
   (i) providing a shrinkable biopolymer fiber as sensor, and
   (ii) observing whether a shrinkage of said fiber occurs,
      wherein a shrinkage of at least 10% with regard to the total length of said fiber is indicative for the presence of a solvent in contact with said fiber.
10. The use of the shrinkable biopolymer fiber of any one of items 3 to 7 or the method of items 8 or 9, wherein the shrinkage starts between 3and 200 seconds after (first) contact with the solvent/after (first) contact of the solvent with said fiber.
11. The use of the shrinkable biopolymer fiber of any one of items 3 to 7 or 10, or the method of any one of items 8 to 10, wherein the shrinkage is concluded to at least 80% after a time range of between 30 and 700 seconds, preferably of between 33 and 550 seconds, and more preferably of between 110 and 250 seconds.
12. The use of the shrinkable biopolymer fiber of any one of items 3 to 7, 10 or 11, or the method of any one of items 8 to 11, wherein the solvent is an aqueous solution, preferably water, or a solution comprising alcohol.
13. The use of the shrinkable biopolymer fiber of any one of items 1 to 7 or 10 to 12, or the method of any one of items 8 to 12, wherein the biopolymer is a silk polypeptide, preferably a recombinant silk polypeptide.
14. The use of the shrinkable biopolymer fiber of any one of items 4 to 7 or 10 to 13, or the method of any one of items 8 or 10 to 13, wherein the product is a fabric, preferably a woven fabric or knitted fabric, apparel, more preferably a garment.
15. The use of the shrinkable biopolymer fiber of any one of items 1 to 7 or 10 to 14, or the method of any one of items 8 or 10 to 14, wherein said fiber is part of a label or hangtag.
16. The use of the shrinkable biopolymer fiber or the method of item 15, wherein the label or hangtag is attached to the product or is part of the product, or wherein the label or hangtag is attached to the packaging or is part of the packaging comprising the product.
17. Use of a shrinkable biopolymer fiber for shaping an object.
18. A method for shaping an object comprising the steps of:
   (i) providing an object comprising or consisting of a shrinkable biopolymer fiber, and
   (ii) contacting said object with a solvent, thereby shaping the object.
19. Use of a shrinkable biopolymer fiber as suture material.
20. Use of a shrinkable biopolymer fiber as wound dressing.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following figures and examples are merely illustrative of the present invention and should not be construed to limit the scope of the invention as indicated by the appended claims in any way.

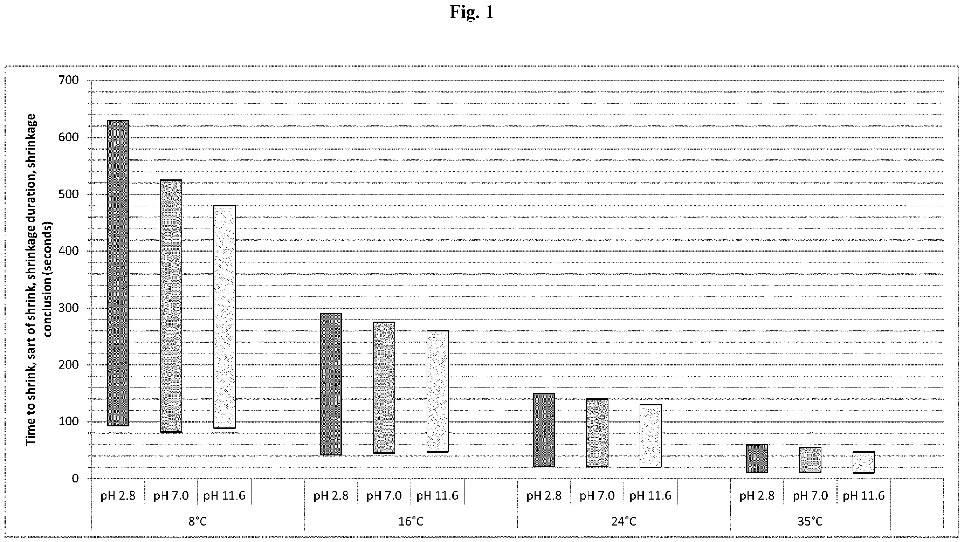

**Figure 1****:** It shows the time to shrink, start of shrinkage, shrinkage duration and shrinkage conclusion of a biopolymer fiber depending on the influence of temperature and pH. The fiber was immersed in buffered aqueous solutions at three different pH values maintaining a constant salt content (pH 2,8 10 mM NaCl, pH 7,0 10 mM NaCl, pH 11,6 10 mM NaCl) at four different temperatures (8°C, 16°C, 24°C and 35°C). The time range between 0 and lowest value of each bar corresponds to the duration between first contact with a solvent/first contact of a solvent with said fiber and start of shrink (time to shrink). The lowest value of each bar corresponds to the starting time of shrinkage (start of shrinkage), the highest value of each bar corresponds the end of the shrinkage process (shrinkage conclusion) and the value between lowest and highest value of each bar corresponds to the time range between start of shrink and the end time/stop of the contraction of the fiber (shrinkage duration).
   It could be demonstrated that the biopolymer fiber has a specific shrinkage behavior which is influenced by temperature. The shrinkage (process) started after a duration after first contact with a solvent/after first contact of a solvent with said fiber and was concluded after a time range. An increase in temperature reduced the duration between first contact of a solvent with said fiber ("time to shrink") and start of the shrinkage. It could further be shown that an increase in temperature reduced the time range in which the shrinkage is concluded ("shrinkage conclusion"). In addition, it could be demonstrated that the biopolymer fiber has a specific shrinkage behavior which is influenced by pH: an increase in pH (towards a more basic pH) reduced the time range in which the shrinkage is concluded ("shrinkage conclusion").

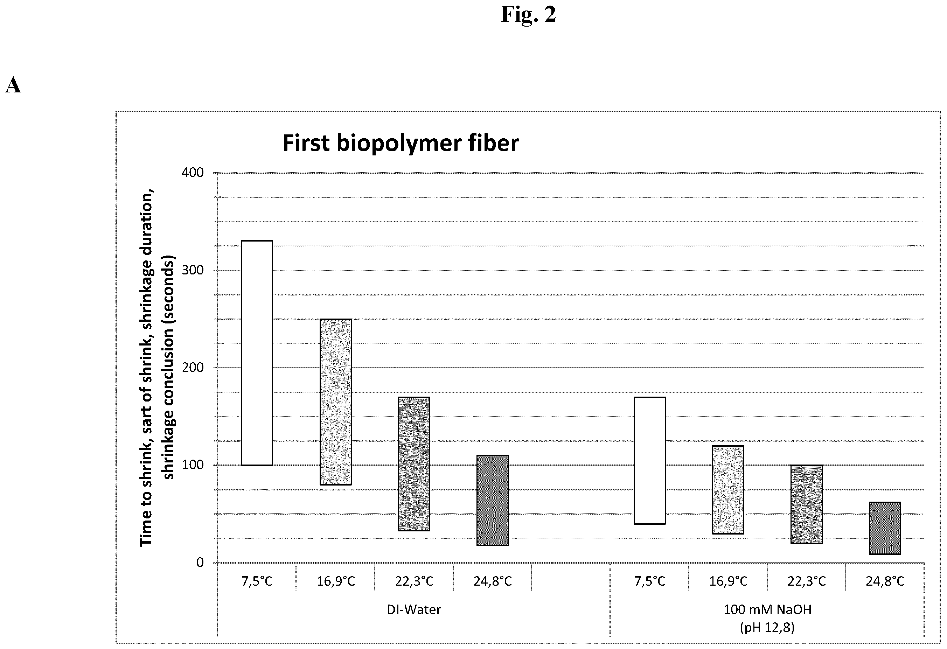

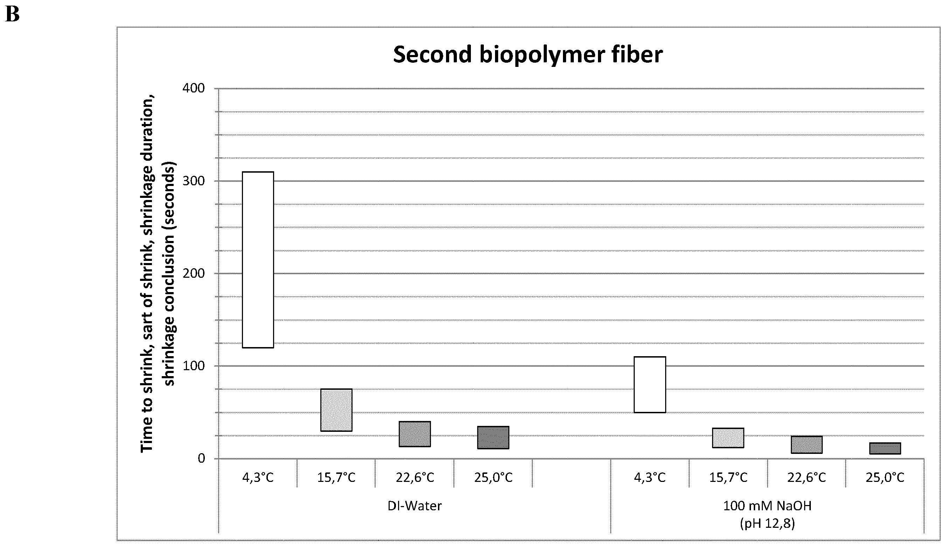

**Figure 2****:** It shows the time to shrink, start of shrinkage, shrinkage duration and shrinkage conclusion of two different biopolymer fibers of varying diameter depending on the influence of temperature and pH.
   The shrinkage behavior (start of shrink and the shrinkage duration) between two different fibers of varying diameters (a first silk biopolymer multifiber with a diameter of approximately 250 µm and a second silk biopolymer fiber with a diameter of approximately 76 µm) were determined: Therefore the first and second biopolymer fiber were contacted with an aqueous solvent. The time range between 0 and lowest value of each bar corresponds to the duration between first contact of a solvent with said fiber and start of shrink (time to shrink). The lowest value of each bar corresponds to the starting time of shrinkage (start of shrinkage), the highest value of each bar corresponds the end of the shrinkage process (shrinkage conclusion) and the value between lowest and highest value of each bar corresponds to time range between start of shrink and end time/stop of the contraction of the fiber (shrinkage duration).
   Figure 2 A represents the shrinkage behavior of the first biopolymer fiber. Figure 2 B represents the shrinkage behavior of the second biopolymer fiber. It could be demonstrated that the biopolymer fiber has a specific shrinkage behavior which is influenced by the diameter of the fiber. The shrinkage process started after a duration after first contact of a solvent with said fiber and was concluded after a time range. A decrease in diameter of the fiber reduced the time to shrink and the shrinkage duration in deionized water and buffered aqueous solution.
   The shrinkage behavior of the biopolymer fiber in deionized water (neutral pH) compared to the shrinkage behavior of the biopolymer fiber in buffered aqueous solution (pH 12,8) shows that an increase in pH (towards a more basic pH) reduced the time to shrink and the shrinkage duration. As the lowest temperature value differs between first and second biopolymer fiber (first biopolymer fiber: 7.5°C, second biopolymer fiber 4.3°C) the results for this lowest temperature value are not directly comparable between first and second biopolymer fiber. It should be noted that a higher temperature results in a reduction of time to shrink and shrinkage duration.

### EXAMPLES

The examples given below are for illustrative purposes only and do not limit the invention described above in any way.

### Example 1: Determination of the starting time of the shrinkage of the fiber after first contact with a solvent and the duration between start of the shrinkage process and end of the shrinkage process

### Definitions:

- contact with solvent: first contact with the solvent
- time to shrink: duration between first contact with a solvent/first contact of a solvent with said fiber and start of shrink
- start of shrink: starting time of the shrinkage/first contraction of the fiber
- shrinkage duration: time range between first contraction of the fiber ("start of shrink") and stop of the contraction of the fiber
- shrinkage conclusion: end time/stop of the contraction of the fiber

In order to determine the starting time of the shrinkage/first contraction of the fiber after first contact with the solvent ("start of shrink") and the time range ("shrinkage duration") between start of shrink and end time/stop of the contraction of the fiber ("shrinkage conclusion") a C₃₂ silk biopolymer multifiber was contacted with an aqueous solvent. The time range between contact with the solvent and start of shrink and as well as the time range between start of the shrink and end of the shrinkage ("shrinkage conclusion") was measured.

Therefore, three C₃₂ silk fibers were immersed into a glass cylinder/measuring cylinder filled with the respective solvent. The fibers were fixed with one end at the top of the glass cylinder. A metal nut fixed at the other end of the fiber served as weight to fully extended the fiber. The timer was set to zero at first contact with the solvent ("contact with solvent"). The time range between contact with solvent and first movement of fiber contraction ("start of shrink") represented the time range "time to shrink". The time range between first contraction of the fiber ("start of shrink") and stop of the contraction of the fiber represented the "shrinkage duration".

The silk biopolymer was composed of 100% C₃₂NR4 silk protein. The silk protein was prepared as described in WO 2006/008163. The protein was then processed into fibers as described in WO 2014/037453. The fiber used for the experiments is a multifilament consisting of 40 monofilaments. The fiber has an overall diameter of approximately 140 µm

The experiments were carried out in triple at four different temperatures (8°C, 16°C, 24°C, 35°C). In a first experiment, the fiber was immersed in buffered aqueous solutions at three different pH values maintaining a constant salt content (pH 2,8 10 mM NaCl, pH 7,0 10 mM NaCl, pH 11,6 10 mM NaCl). In a second experiment, the fiber was immersed in buffered aqueous solutions at different salt contents maintaining a constant pH value (50 mM NaCl pH 7,0, 100 mM NaCl pH 7,0, 200 mM NaCl pH 7,0).

The results are shown in **Figure 1****.** It could be demonstrated that the biopolymer fiber has a specific shrinkage behavior which is influenced by temperature. The shrinkage (process) started after a duration after first contact with a solvent/after first contact of a solvent with said fiber and was concluded after a time range. An increase in temperature reduced the duration between first contact of a solvent with said fiber ("time to shrink") and start of the shrinkage. It could further be shown that an increase in temperature reduced the time range in which the shrinkage is concluded ("shrinkage conclusion"). In addition, it could be demonstrated that the biopolymer fiber has a specific shrinkage behavior which is influenced by pH: an increase in pH (towards a more basic pH) reduced the time range in which the shrinkage is concluded ("shrinkage conclusion").

### Example 2: Influence of the thickness of the fiber on the shrinkage behaviour

In order to determine the different shrinkage behavior (start of shrink and shrinkage duration) between two different fibers of varying diameters a first silk biopolymer multifiber (with a diameter of approximately 250 µm) and a second silk biopolymer fiber (with a diameter of approximately 76 µm) were contacted with an aqueous solvent. The time range between contact with the solvent and start of shrink and as well as shrinkage conclusion were measured.

Therefore the first and the second silk biopolymer fiber were immersed into a glass cylinder / measuring cylinder filled with the respective solvent. The biopolymer fibers were fixed with one end at the top of the glass cylinder. A metal nut fixed at the other end of the fiber served as weight to fully extended the fiber. The timer was set to zero at first contact with the solvent. The time range between contact with solvent and first movement of fiber contraction ("start of shrink") represents time range "time to shrink". The time range between first contraction of the fiber ("start of shrink") and stop of the contraction of the fiber represents the shrinkage duration.

The first and second silk biopolymer fiber were composed of 100% C₃₂NR4 silk protein. The silk protein was prepared as described in WO 2006/008163. The protein was then processed into fibers as described in WO 2014/037453. The first biopolymer fiber used for the experiments was a multifilament comprising three multifilaments, each multifilament consisting of 40 monofilaments. The three multifilaments were twisted into a yarn which comprises three multifilaments, each multifilament consisting of 40 monofilaments. The resulting fiber has an overall diameter of approximately 250 µm. The second biopolymer fiber used for the experiments was a multifilament consisting of 30 monofilaments with a diameter of approximately 76 µm.

The experiments were carried out at different temperatures (first biopolymer fiber: 7.5°C, 16.9°C, 22.3°C, 24.8°C; second biopolymer fiber: 4.3°C, 15.7°C, 22.63°C, 25.0°C). In a first experiment the first and second biopolymer fiber were immersed in deionized water. In a second experiment the first and second biopolymer fiber were immersed in a buffered aqueous solution (pH 12,8 100 mM NaCl).

The results are shown in Figure 2. Figure 2 A represents the shrinkage behavior of the first biopolymer fiber. Figure 2 B represents the shrinkage behavior of the second biopolymer fiber. It could be demonstrated that the biopolymer fibers have a specific shrinkage behavior which is influenced by the diameter of the fiber. The shrinkage process started after a duration after first contact of a solvent with said fiber and was concluded after a time range. A decrease in diameter of the fiber reduced the time to shrink and the shrinkage duration in both experiments (deionized water and buffered aqueous solution).

The shrinkage behavior of the biopolymer fiber in deionized water (neutral pH) compared to the shrinkage behavior of the biopolymer fiber in buffered aqueous solution (pH 12,8) shows that an increase in pH (towards a more basic pH) reduced the time to shrink and the shrinkage duration. As the lowest temperature value differs between first and second biopolymer fiber (first biopolymer fiber: 7.5°C, second biopolymer fiber 4.3°C) the results for this lowest temperature value are not directly comparable. It should be noted that a higher temperature results in a reduction of time to shrink and shrinkage duration.

## Claims

1. Use of a shrinkable biopolymer fiber for shaping an object.

2. The use of claim 1, wherein said fiber shows a shrinkage of at least 10% with regard to its total length after contact with a solvent.

3. The use of claim 2, wherein the solvent is an aqueous solution, preferably water, or a solution comprising alcohol.

4. The use of any one of claims 1 to 3, wherein the shaping allows the manufacture of a tailored and/or customized object.

5. The use of any one of claims 1 to 4, wherein the object is a fabric, preferably a garment or apparel, or footwear.

6. The use of any one of claims 1 to 5, wherein the biopolymer is a silk polypeptide, preferably a recombinant silk polypeptide.

7. A method for shaping an object comprising the steps of:
(i) contacting an object comprising or consisting of a shrinkable biopolymer fiber, with a solvent, and
(ii) observing shrinkage of the shrinkable biopolymer fiber, thereby shaping the object.

8. The method of claim 7, wherein said fiber shows a shrinkage of at least 10% with regard to its total length after contact with a solvent.

9. The method of claims 7 or 8, wherein said object is contacted with the solvent by dropping the solvent onto said object, by dipping said object into the solvent, or by spraying the solvent onto said object.

10. The method of any one of claims 7 to 9, wherein the solvent is an aqueous solution, preferably water, or a solution comprising alcohol.

11. The method of any one of claims 7 to 10, wherein the shaping allows the manufacture of a tailored and/or customized object.

12. The method of any one of claims 7 to 11, wherein the object is a fabric, preferably a garment or apparel, or footwear.

13. The method any one of claims 7 to 12, wherein the biopolymer is a silk polypeptide, preferably a recombinant silk polypeptide.

14. A shrunk object obtainable by the method of any one of claim 7 to 13.
